# EUROPEAN PATENT SPECIFICATION

(11) **EP 2 989 988 B1**
(45) Date of publication and mention of the grant of the patent: **04.10.2017**
(21) Application number: 15165354.0
(22) Date of filing: 28.04.2015
(51) Int. Cl.: A61B 8/08, A61B 8/00, G06T 7/00

(54) **ULTRASOUND IMAGE DISPLAY APPARATUS AND METHOD OF DISPLAYING ULTRASOUND IMAGE**
ULTRASCHALLBILDANZEIGEVORRICHTUNG UND VERFAHREN ZUR ANZEIGE EINES ULTRASCHALLBILDES
APPAREIL D'AFFICHAGE D'IMAGE ULTRASONORE ET PROCEDE D'AFFICHAGE D'UNE IMAGE ULTRASONORE

(30) Priority: 29.08.2014 US 201462043773 P; 17.10.2014 KR 20140141201
(43) Date of publication of application: 02.03.2016
(73) Proprietor: Samsung Medison Co., Ltd., Hongcheon-gun, Gangwon-do (KR)
(72) Inventor: Lee, Kwang-hee, Gangwon-do (KR)
(74) Representative: D'Halleweyn, Nele Veerle Trees Gertrudis

(56) References cited:
- EP-A1- 2 570 081
- WO-A1-2010/073178
- US-A1- 2011 274 330

## Description

### BACKGROUND

### 1. Field

One or more embodiments of the present invention relate to an ultrasound image display apparatus and a method of displaying an ultrasound image, and more particularly, to an ultrasound image display apparatus and a method of displaying an ultrasound image, by which a variation, over time, in a target included in an object is easily diagnosed.

### 2. Description of the Related Art

Ultrasound diagnosis apparatuses irradiate an ultrasound signal generated by a transducer of a probe to an object and receives information regarding an echo signal reflected from the object, thereby obtaining an image of a part inside the object. In particular, ultrasound diagnosis apparatuses are used for medical purposes, such as observation of the inside of an object, detection of foreign substances inside the object, and diagnosis of damage thereof. Such ultrasound diagnosis apparatuses have various advantages, including stability, real-time display, and safety because there is no exposure to radiation, compared to X-ray apparatuses, and thus, the ultrasound diagnosis apparatuses are commonly used together with other image diagnosis apparatuses.

An ultrasound image display apparatus and a method of displaying an ultrasound image, by which ultrasound data acquired by an ultrasound diagnosis apparatus may be efficiently displayed, are required.

US 2011/274330 A1 discloses a non-invasive medical imaging system including an imaging scanner capable of generating an image signal from a subject under observation inside the imaging scanner, a signal processing system in communication with the imaging scanner and a data storage unit in communication with the signal processing system. A first image corresponding to a tissue region of the subject is stored in the data storage unit and the signal processing system generates a second image encoding the tissue region of the subject by performing a reconstruction based on the imaging signal, wherein the imaging signal is acquired at a later time than the first image. The signal processing system then provides a registered first image by registering the first image to the second image via a transformation in a space of diffeomorphism and the signal processing system computes a difference image between the second image and the registered first image.

WO 2010/073178 A1 discloses a medical imaging system including at least one controller which is configured to receive first image information corresponding with one or more images acquired at a first time and second image information corresponding with one or more images acquired at another time. The controller then determines whether first coordinate information corresponding to one or more locations in the first image information has changed relative to second coordinate information corresponding to one or more locations in the second image information. The controller then highlights the first image information based upon the result of the determination.

### SUMMARY

One or more exemplary embodiments include an ultrasound image display apparatus and a method of displaying an ultrasound image, by which a variation, over time, in an object is easily diagnosed. In detail, one or more exemplary embodiments include an ultrasound image display apparatus and a method of displaying an ultrasound image, by which, when an object needs to be observed at time intervals, a user may easily observe changes in the object at subsequent time points.

Additional aspects will be set forth in part in the description which follows and, in part, will be apparent from the description, or may be learned by practice of the presented embodiments.

According to one or more embodiments of the present invention, an ultrasound image display apparatus includes an image processor which acquires respective pieces of ultrasound data for a plurality of time points, which represent an object including at least one target at a plurality of different time points, and acquires first information representing a change in the at least one target at the plurality of different time points, based on a correspondence between the respective pieces of ultrasound data for the plurality of time points; and a display which displays a screen image including a diagnosis image that shows the first information.

The diagnosis image may be an ultrasound image displayed so that states of the at least one target at the plurality of different time points may be distinguished from one another.

The respective pieces of ultrasound data for the plurality of time points may include first ultrasound data acquired by scanning the object at a first time point, and second ultrasound data acquired by scanning the object at a second time point.

The diagnosis image may be an ultrasound image in which a first target image representing the at least one target based on the first ultrasound data and a second target image representing the at least one target based on the second ultrasound data are overlappingly displayed.

The first target image and the second target image may be distinguishable from each other when displayed in the diagnosis image.

A difference between the first target image and the second target image may be highlighted in the diagnosis image.

The image processor may acquire a first size of the at least one target based on the first ultrasound data and a second size of the at least one target based on the second ultrasound data.

The display may further display at least one selected from size information for the first size, size information for the second size, and information representing a size change of the at least one target, which are acquired based on the first size and the second size.

The display may further display information about a size change of the at least one target over time at the plurality of different time points.

The image processor may acquire second registered data by transforming the second ultrasound data to align with the first ultrasound data, and the screen image may further include a first image based on the first ultrasound data and a second image based on the second registered data.

The image processor may respectively segment a plurality of separate areas included in the first ultrasound data and a plurality of separate areas included in the second ultrasound data, respectively detect a reference point of each of the plurality of separate areas included in the first ultrasound data and each of the plurality of separate areas included in the second ultrasound data, match a first reference point from among the reference points included in the first ultrasound data with a second reference point from among the reference points included in the second ultrasound data, and perform image registration with respect to the first ultrasound data and the second ultrasound data, based on the matching between the first reference point and the second reference point.

The image processor may match the first reference point with the second reference point by using an iterative closest point (ICP).

The image processor may detect volume information about each of the plurality of separate areas and match the first reference point with the second reference point, based on the volume information.

The image processor may match the first reference point with the second reference point by applying a weight to each of the reference points based on the volume information.

The image processor may perform image registration with respect to the first ultrasound data and the second ultrasound data by using at least one selected from mutual information, a correlation coefficient, ratio-image uniformity, and partitioned intensity uniformity.

The image processor may perform image registration with respect to the first ultrasound data and the second ultrasound data via a random sample consensus (RANSAC).

The image processor may respectively segment a plurality of separate areas included in the first ultrasound data and a plurality of separate areas included in the second ultrasound data, and detect at least one of the plurality of separate areas included in each of the first ultrasound data and the second ultrasound data, as at least one target area that is a separate area for the at least one target.

The image processor may detect a size of each of the plurality of separate areas and detect the target area based on the size.

The object may be an ovary, and the at least one target may include a follicle, in which ovulation is induced, from among follicles included in the ovary.

The object may be a part of the abdomen including a womb, and the at least one target may include at least one tumor generated in at least one part of within a womb and an outside womb.

The ultrasound image display apparatus may further include a memory which stores the respective pieces of ultrasound data for the plurality of time points.

The screen image may include respective ultrasound images for a plurality of time points, which is obtained based on the respective pieces of ultrasound data for the plurality of time points, and the respective ultrasound images for the plurality of time points may be arranged in the order of time points at which the object is scanned.

The first information may represent a change in at least one selected from the size, position, and number of the at least one target at the plurality of different time points.

The image screen may further include target change numeric information that numerically represents a change in at least one selected from the size, position, and number of the at least one target.

The target change numeric information may include a value of at least one selected from an area, a volume, a long-axis length, a short-axis length, a radius, a diameter, and a circumference that represent the size of the at least one target.

The target change numeric information may include a variation in the value of the at least one selected from the area, the volume, the long-axis length, the short-axis length, the radius, the diameter, and the circumference of the at least one target.

The image processor acquires respective ultrasound images for a plurality of time points based on the respective pieces of ultrasound data for the plurality of time points and sets a weight for each of the respective ultrasound images for the plurality of time points. The diagnosis image is an image in which respective ultrasound images for the plurality of time points for each of which the weight is set are overlapped with one another and displayed.

The ultrasound image display apparatus may further include a communicator which receives the respective pieces of ultrasound data for the plurality of time points from an external source.

According to one or more embodiments of the present invention, a method of displaying an ultrasound image includes the features of claim 14.

### BRIEF DESCRIPTION OF THE DRAWINGS

These and/or other aspects will become apparent and more readily appreciated from the following description of the embodiments, taken in conjunction with the accompanying drawings in which:
FIG. 1 is a block diagram showing a configuration of an ultrasound diagnosis apparatus according to an embodiment of the present invention;
FIG. 2 is a block diagram showing a configuration of a wireless probe according to an embodiment;
FIG. 3 is a cross-sectional view of an object which is to be diagnosed in an embodiment of the present invention;
FIG. 4A illustrates an example of a normal ovary;
FIG. 4B illustrates an example of a polycystic ovary;
FIG. 5A is a block diagram of an ultrasound image display apparatus according to an embodiment; FIG. 5B is a block diagram of an ultrasound image display apparatus according to another embodiment;
FIG. 6 illustrates an example of ultrasound data that is acquired by ultrasound image display apparatuses according to some embodiments;
FIG. 7 illustrates ultrasound images acquired from such ultrasound data as FIG. 6;
FIG. 8 illustrates image registration that is performed by image processors of ultrasound image display apparatuses according to some embodiments;
FIG. 9 illustrates a diagnosis image that is acquired by image processors of ultrasound image display apparatuses according to some embodiments;
FIG. 10 illustrates a diagnosis image that is acquired by image processors of ultrasound image display apparatuses according to some embodiments;
FIG. 11 illustrates a screen of a display of ultrasound image display apparatuses according to some embodiments;
FIGS. 12 and 13 illustrate processes in which am image processor of an ultrasound image display apparatus according to an embodiment acquires a diagnosis image via image registration;
FIGS. 14-17 illustrate image registration that is performed by using an iterative closest point (ICP);
FIG. 18 illustrates ultrasound data processing for image registration that is performed by an image processor of an ultrasound image display apparatus according to an embodiment;
FIG. 19A illustrates a screen image that is displayed by an ultrasound image display apparatus according to an embodiment;
FIG. 19B illustrates a screen image that is displayed by an ultrasound image display apparatus according to an embodiment;
FIG. 20A illustrates a screen image that is displayed by an ultrasound image display apparatus according to an embodiment;
FIG. 20B illustrates a screen image that is displayed by an ultrasound image display apparatus according to an embodiment;
FIG. 21A illustrates a screen image that is displayed by an ultrasound image display apparatus according to an embodiment;
FIG. 21B illustrates a screen image that is displayed by an ultrasound image display apparatus according to an embodiment;
FIG. 22 illustrates a screen image that is displayed by an ultrasound image display apparatus according to an embodiment; and
FIG. 23 is a flowchart of an ultrasound image displaying method according to an embodiment.

### DETAILED DESCRIPTION

All terms including descriptive or technical terms which are used herein should be construed as having meanings that are obvious to one of ordinary skill in the art. However, the terms may have different meanings according to the intention of one of ordinary skill in the art, precedent cases, or the appearance of new technologies. Also, some terms may be arbitrarily selected by the applicant, and in this case, the meaning of the selected terms will be described in detail in the detailed description of the invention. Thus, the terms used herein have to be defined based on the meaning of the terms together with the description throughout the specification.

When a part "includes" or "comprises" an element, unless there is a particular description contrary thereto, the part can further include other elements, not excluding the other elements. In addition, terms such as "... unit", "... module", or the like refer to units that perform at least one function or operation, and the units may be implemented as hardware or software or as a combination of hardware and software.

Throughout the specification, an "ultrasound image" refers to an image of an object, which is obtained using ultrasound waves. Furthermore, an "object" may be a human, an animal, or a part of a human or animal. For example, the object may be an organ (e.g., the liver, the heart, the womb, the brain, a breast, or the abdomen), a blood vessel, or a combination thereof. Also, the object may be a phantom. The phantom means a material having a density, an effective atomic number, and a volume that are approximately the same as those of an organism.

Throughout the specification, a "user" may be, but is not limited to, a medical expert, for example, a medical doctor, a nurse, a medical laboratory technologist, or a medical imaging expert, or a technician who repairs medical apparatuses.

Embodiments of the invention now will be described more fully hereinafter with reference to the accompanying drawings, in which illustrative embodiments of the invention are shown.

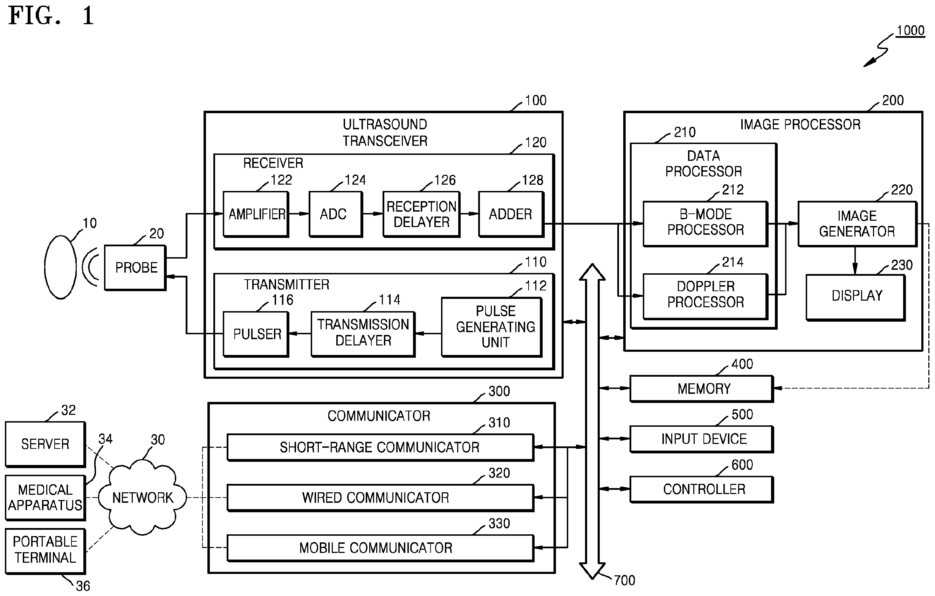

FIG. 1 is a block diagram showing a configuration of an ultrasound diagnosis apparatus 1000 according to an embodiment of the present invention. Referring to FIG. 1, the ultrasound diagnosis apparatus 1000 may include a probe 20, an ultrasound transceiver 100, an image processor 200, a communication module 300, a display 300, a memory 400, an input device 500, and a controller 600, which may be connected to one another via buses 700.

The ultrasound diagnosis apparatus 1000 may be a cart type apparatus or a portable type apparatus. Examples of portable ultrasound diagnosis apparatuses may include, but are not limited to, a picture archiving and communication system (PACS) viewer, a smartphone, a laptop computer, a personal digital assistant (PDA), and a tablet PC.

The probe 20 transmits ultrasound waves to an object 10 in response to a driving signal applied by the ultrasound transceiver 100 and receives echo signals reflected by the object 10. The probe 20 includes a plurality of transducers, and the plurality of transducers oscillate in response to electric signals and generate acoustic energy, that is, ultrasound waves. Furthermore, the probe 20 may be connected to the main body of the ultrasound diagnosis apparatus 1000 by wire or wirelessly.

A transmitter 110 supplies a driving signal to the probe 20. The transmitter 1110 includes a pulse generator 112, a transmission delaying unit 114, and a pulser 116. The pulse generator 112 generates pulses for forming transmission ultrasound waves based on a predetermined pulse repetition frequency (PRF), and the transmission delaying unit 114 delays the pulses by delay times necessary for determining transmission directionality. The pulses which have been delayed correspond to a plurality of piezoelectric vibrators included in the probe 20, respectively. The pulser 116 applies a driving signal (or a driving pulse) to the probe 20 based on timing corresponding to each of the pulses which have been delayed.

A receiver 120 generates ultrasound data by processing echo signals received from the probe 20. The receiver 120 may include an amplifier 122, an analog-to-digital converter (ADC) 124, a reception delaying unit 126, and a summing unit 128. The amplifier 122 amplifies echo signals in each channel, and the ADC 124 performs analog-to-digital conversion with respect to the amplified echo signals. The reception delaying unit 126 delays digital echo signals output by the ADC 1124 by delay times necessary for determining reception directionality, and the summing unit 128 generates ultrasound data by summing the echo signals processed by the reception delaying unit 1126. Also, according to embodiments of the present invention, the receiver 120 may not include the amplifier 122. In other words, if the sensitivity of the probe 20 or the capability of the ADC 124 to process bits is enhanced, the amplifier 122 may be omitted.

The image processor 200 generates an ultrasound image by scan-converting ultrasound data generated by the ultrasound transceiver 100 and displays the ultrasound image. The ultrasound image may be not only a grayscale ultrasound image obtained by scanning an object in an amplitude (A) mode, a brightness (B) mode, and a motion (M) mode, but also a Doppler image showing a movement of an object via a Doppler effect. The Doppler image may be a blood flow Doppler image showing flow of blood (also referred to as a color Doppler image), a tissue Doppler image showing a movement of tissue, or a spectral Doppler image showing a moving speed of an object as a waveform.

A B mode processor 212 extracts B mode components from ultrasound data and processes the B mode components. An image generator 220 may generate an ultrasound image indicating signal intensities as brightness based on the extracted B mode components.

Similarly, a Doppler processor 214 may extract Doppler components from ultrasound data, and the image generator 220 may generate a Doppler image indicating a movement of an object as colors or waveforms based on the extracted Doppler components.

According to an embodiment of the present invention, the image generator 220 may generate a three-dimensional (3D) ultrasound image via volume-rendering with respect to volume data and may also generate an elasticity image by imaging deformation of the object 10 due to pressure. Furthermore, the image generator 220 may display various pieces of additional information in an ultrasound image by using text and graphics. In addition, the generated ultrasound image may be stored in the memory 400.

A display 230 displays the generated ultrasound image. The display 230 may display not only an ultrasound image, but also various pieces of information processed by the ultrasound diagnosis apparatus 1000 on a screen image via a graphical user interface (GUI). In addition, the ultrasound diagnosis apparatus 1000 may include two or more displays 230 according to embodiments of the present invention.

The communication module 300 is connected to a network 30 by wire or wirelessly to communicate with an external device or a server. The communication module 300 may exchange data with a hospital server or another medical apparatus in a hospital, which is connected thereto via a PACS. Furthermore, the communication module 300 may perform data communication according to the digital imaging and communications in medicine (DICOM) standard.

The communication module 300 may transmit or receive data related to diagnosis of an object, e.g., an ultrasound image, ultrasound data, and Doppler data of the object, via the network 30 and may also transmit or receive medical images captured by another medical apparatus, e.g., a computed tomography (CT) apparatus, a magnetic resonance imaging (MRI) apparatus, or an X-ray apparatus. Furthermore, the communication module 300 may receive information about a diagnosis history or medical treatment schedule of a patient from a server and utilizes the received information to diagnose the patient. Furthermore, the communication module 300 may perform data communication not only with a server or a medical apparatus in a hospital, but also with a portable terminal of a medical doctor or patient.

The communication module 300 is connected to the network 30 by wire or wirelessly to exchange data with a server 32, a medical apparatus 34, or a portable terminal 36. The communication module 300 may include one or more components for communication with external devices. For example, the communication module 1300 may include a local area communication module 310, a wired communication module 320, and a mobile communication module 330.

The local area communication module 310 refers to a module for local area communication within a predetermined distance. Examples of local area communication techniques according to an embodiment of the present invention may include, but are not limited to, wireless LAN, Wi-Fi, Bluetooth, ZigBee, Wi-Fi Direct (WFD), ultra wideband (UWB), infrared data association (IrDA), Bluetooth low energy (BLE), and near field communication (NFC).

The wired communication module 320 refers to a module for communication using electric signals or optical signals. Examples of wired communication techniques according to an embodiment of the present invention may include communication via a twisted pair cable, a coaxial cable, an optical fiber cable, and an Ethernet cable.

The mobile communication module 330 transmits or receives wireless signals to or from at least one selected from a base station, an external terminal, and a server on a mobile communication network. The wireless signals may be voice call signals, video call signals, or various types of data for transmission and reception of text/multimedia messages.

The memory 400 stores various data processed by the ultrasound diagnosis apparatus 1000. For example, the memory 400 may store medical data related to diagnosis of an object, such as ultrasound data and an ultrasound image that are input or output, and may also store algorithms or programs which are to be executed in the ultrasound diagnosis apparatus 1000.

The memory 400 may be any of various storage media, e.g., a flash memory, a hard disk drive, EEPROM, etc. Furthermore, the ultrasound diagnosis apparatus 1000 may utilize web storage or a cloud server that performs the storage function of the memory 400 online.

The input device 500 refers to a means via which a user inputs data for controlling the ultrasound diagnosis apparatus 1000. The input device 500 may include hardware components, such as a keypad, a mouse, a touch panel, a touch screen, and a jog switch. However, embodiments of the present invention are not limited thereto, and the input device 1600 may further include any of various other input units including an electrocardiogram (ECG) measuring module, a respiration measuring module, a voice recognition sensor, a gesture recognition sensor, a fingerprint recognition sensor, an iris recognition sensor, a depth sensor, a distance sensor, etc.

The controller 600 may control all operations of the ultrasound diagnosis apparatus 1000. In other words, the controller 600 may control operations among the probe 20, the ultrasound transceiver 100, the image processor 200, the communication module 300, the memory 400, and the input device 500 shown in FIG. 1.

All or some of the probe 20, the ultrasound transceiver 100, the image processor 200, the communication module 300, the memory 400, the input device 500, and the controller 600 may be implemented as software modules. However, embodiments of the present invention are not limited thereto, and some of the components stated above may be implemented as hardware modules. Furthermore, at least one selected from the ultrasound transceiver 100, the image processor 200, and the communication module 300 may be included in the controller 1700. However, embodiments of the present invention are not limited thereto.

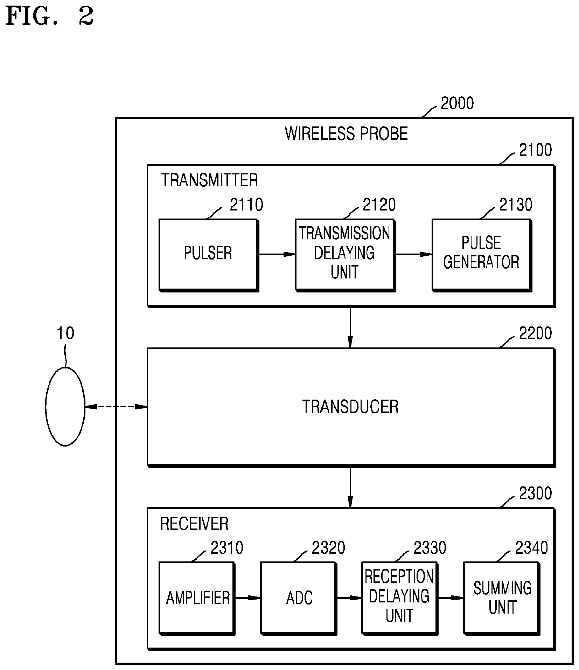

FIG. 2 is a block diagram showing a configuration of a wireless probe 2000 according to an embodiment. As described above with reference to FIG. 1, the wireless probe 2000 may include a plurality of transducers, and, according to embodiments of the present invention, may include some or all of the components of the ultrasound transceiver 100 shown in FIG. 1.

The wireless probe 2000 according to the embodiment shown in FIG. 2 includes a transmitter 2100, a transducer 2200, and a receiver 2300. Since descriptions thereof are given above with reference to FIG. 1, detailed descriptions thereof will be omitted here. In addition, according to embodiments of the present invention, the wireless probe 2000 may selectively include a reception delaying unit 2330 and a summing unit 2340.

The wireless probe 2000 may transmit ultrasound signals to the object 10, receive echo signals from the object 10, generate ultrasound data, and wirelessly transmit the ultrasound data to the ultrasound diagnosis apparatus 1000 shown in FIG. 1.

An ultrasound image display apparatus according to an embodiment of the present invention includes all medical imaging apparatuses capable of processing, generating, and/or displaying an ultrasound image by using ultrasound data that is acquired by at least one selected from the ultrasound diagnosis apparatus 1000 of FIG. 1 and the wireless probe 2000 of FIG. 2.

The ultrasound image display apparatus according to an embodiment of the present invention displays a first ultrasound image including first information that represents a change in at least one selected from the size, position, and number of at least one target included in an object, by using ultrasound data acquired by performing an ultrasound scan on the object.

An object used herein is a body part that needs to be examined in connection with gynecological disease, and thus may be a part of the lower abdomen of a woman. In detail, the object may be an ovary including at least one follicle. Alternatively, the object may be a womb including at least one tumor or a part of the lower abdomen of a woman including at least one tumor. Alternatively, the object may be a specific body part or specific organ including at least one abnormal tissue.

There are times that at least one target included in the object needs to be monitored in connection with gynecological disease. In detail, there are times to scan the object at a plurality of time points and observe how much the object changes during a period of time including the plurality of time points. For example, to cure the polycystic ovary syndrome, a change in an ovary needs to be monitored at regular time intervals during a predetermined period of time. As another example, when a womb has a tumor such as a myoma, a user needs to observe a change in the tumor at regular time intervals and determine whether to cure the tumor. Moreover, when an abnormal tissue that needs monitoring exists, a user needs to observe a change in the abnormal tissue at regular time intervals and determine whether to cure the abnormal tissue.

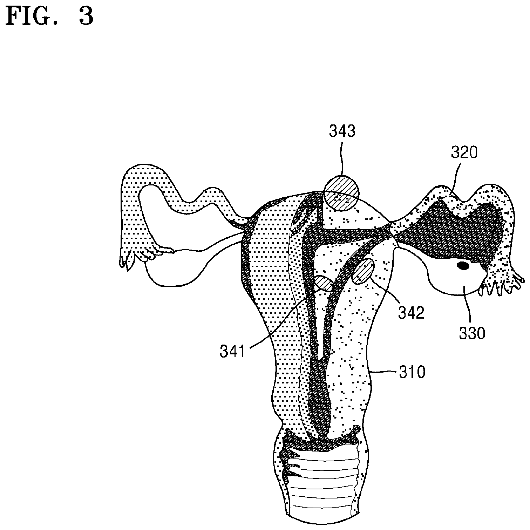

FIG. 3 is a cross-sectional view of an object which is to be diagnosed in an embodiment of the present invention.

Referring to FIG. 3, a womb 310 exists in the lower abdomen of a woman. An ovary 330 is connected to the womb 310 via a fallopian tube 320 included in the womb 310. The ovary 330 includes several follicles and releases one enlarged follicle from among the several follicles according to an ovulation cycle (ovulation). However, if an enlarged follicle fails to be released from an ovary and is left in an ovary, a cyst is generated. When ovulation does not occur, menstruation may be irregular, causing sterility. Thus, to determine whether an ovary, which is set as an object, and ovulation of the ovary are normal, the ovary needs to be observed at a plurality of different time points. In this case, an object is the ovary, and a target may be at least one follicle included in the ovary.

A tumor, such as a myoma, an abnormal tissue, or the like may be generated within the womb 310. Such a tumor or abnormal tissue does not need an action such as an urgent surgery, in contrast with a cancer tissue that is a malignant tumor. However, such a tumor or abnormal tissue may turn to a woman disease such as sterility, and thus there is a need to observe how the tumor or abnormal tissue changes at subsequent time points via monitoring.

In detail, myomas that may be generated in a body part adjacent to the womb 310 include a submucous myoma 341 generated within a uterine cavity that is the inside of the womb 310, an intramural myoma 342 generated outside the uterine cavity, and a subserous myoma 343 generated on a serous membrane that is the outside of the womb 310. In this case, the object may be the lower abdomen including the womb 310, and the target may be a specific myoma.

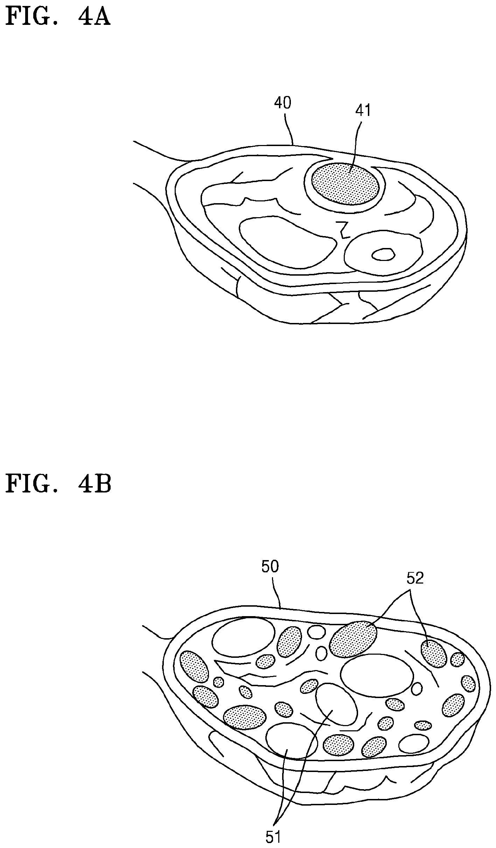

As described above, when at least one target included in an object needs to be monitored over time or a change in the at least one target needs to be observed over time, the ultrasound image display apparatus according to an embodiment of the present invention enables a user to easily ascertain and diagnose changes in the target at a plurality of different time points, thereby increasing user convenience. The ultrasound image display apparatus according to an embodiment of the present invention will now be described in detail with reference to FIGS. 4A-22.

A case where the object is an ovary including at least one follicle and the target is a follicle included in the ovary will now be described as an example. In detail, the ultrasound diagnosis apparatus 1000 of FIG. 1 may acquire ultrasound data about the ovary by scanning the object, namely, the ovary, and a user may diagnose the ovary based on the acquired ultrasound data.

FIG. 4A illustrates an example of a normal ovary 40.

Referring to FIG. 4A, the normal ovary 40 includes numerous primordial follicles (not shown). When a menstrual cycle starts, a plurality of primordial follicles from among the numerous primordial follicles start growing. In the case of human being, about 6-12 primordial follicles start growing. Only one follicle is selected as a dominant follicle 41 from among the plurality of primordial follicles, and the dominant follicle 41 is completely grown and then released.

A polycystic ovary syndrome (PCOS) is a disease in which more follicles than a normal number of follicles are grown within an ovary or follicles are not grown enough to release their ova even when many follicles are grown. The PCOS may cause sterility. For example, when the object is a human and at least 12 follicles each having a size of 2-9 mm are grown within an ovary of the human, the human may have the PCOS.

FIG. 4B illustrates an example of a polycystic ovary 50.

Referring to FIG. 4B, compared to the normal ovary 40 of FIG. 4A, the polycystic ovary 50 includes a plurality of grown follicles 51. In the polycystic ovary 50, follicles that are not released may form cysts 52.

In the case of the PCOS, ovulation may be induced by giving a medicine to a patient so that only one of the plurality of grown follicles 51 is released. A follicle for which ovulation is induced will be hereinafter referred to as a selected follicle. The selected follicle may be at least one of the plurality of grown follicles 51. A diagnosis of whether the selected follicle grows normally during ovulation induction may be necessary.

To diagnose whether the selected follicle grows normally, the size of the selected follicle needs to be monitored over time. A part of the object that needs to be monitored for changes over time, such as, the selected follicle, will be hereinafter referred to as a target. Accordingly, at least one follicle included in an ovary will now be referred to at least one target.

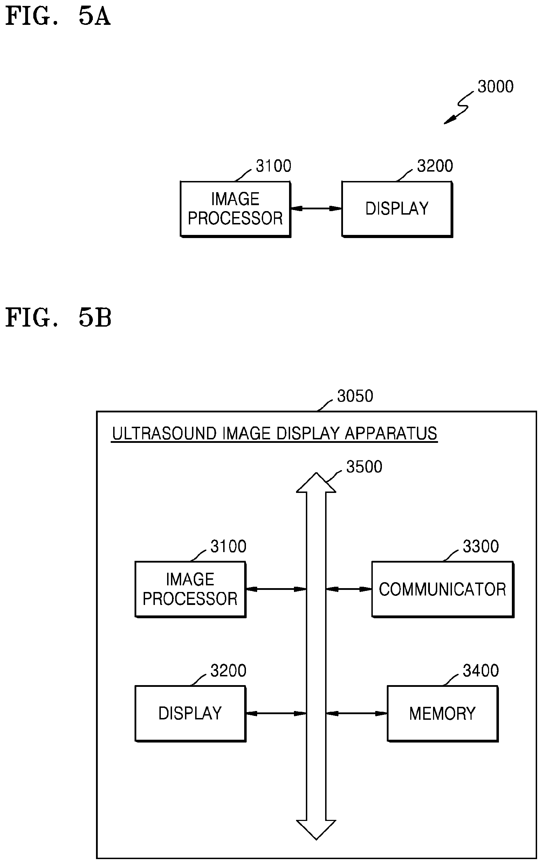

FIG. 5A is a block diagram of an ultrasound image display apparatus 3000 according to an embodiment. The ultrasound image display apparatus 3000 of FIG. 5A may be included in the ultrasound diagnosis apparatus 1000 of FIG. 1. Alternatively, the ultrasound image display apparatus 3000 of FIG. 5A may be included in the medical apparatus 34 or the portable terminal 36 connected to the ultrasound diagnosis apparatus 100 via the network 30. The ultrasound image display apparatus 3000 may be any imaging apparatus capable of acquiring, processing, and displaying an ultrasound image. Accordingly, although not described individually, the above description may be applied to several components included in the ultrasound image display apparatus 3000 of FIG. 5A.

Referring to FIG. 5A, the ultrasound image display apparatus 3000 includes an image processor 3100 and a display 3200.

The image processor 3100 acquires respective pieces of ultrasound data for a plurality of time points that represent an object including at least one target at a plurality of different time points. The image processor 3100 also acquires first information representing a change in the at least one target at the plurality of different time points, based on a correspondence between the acquired respective pieces of ultrasound data for the plurality of time points. The first information may include information that represents a change in at least one selected from the size, position, and number of the at least one target at the plurality of different time points.

In detail, the image processor 3100 acquires a plurality of pieces of ultrasound data corresponding to a plurality of time points by respectively scanning an object including at least one target at a plurality of different time points. The image processor 3100 also acquires first information that represents a change in at least one selected from the size, position, and number of the at least one target at the plurality of different time points, by performing image registration with respect to the respective pieces of ultrasound data for the plurality of time points. The object may be an ovary, and the target may be a follicle. In detail, the target may be a follicle that needs to be monitored over time, such as, the aforementioned selected follicle.

For example, as for a patient having the PCOS, a follicle for which ovulation is induced is set as a target, and it is necessary to monitor whether the selected follicle grows normally during an ovulation cycle. In the aforementioned example, the image processor 3100 acquires first information that represents a change in the target during a predetermined period of time included in the ovulation cycle.

In detail, the first information may be an ultrasound image representing a change in the state of the target that includes changes of the size, position, and number of the target. When the first information is an ultrasound image, the first information may be a first ultrasound image. In detail, the first information may be a first ultrasound image acquired by performing image registration with respect to the respective pieces of ultrasound data for the plurality of time points.

The first information may include numerical values that numerically represent the changes of the size, position, and number of the target. In detail, the first information may be a numerical value that represents a change in the target that is acquired by a registered image obtained by performing image registration with respect to the respective pieces of ultrasound data for the plurality of time points.

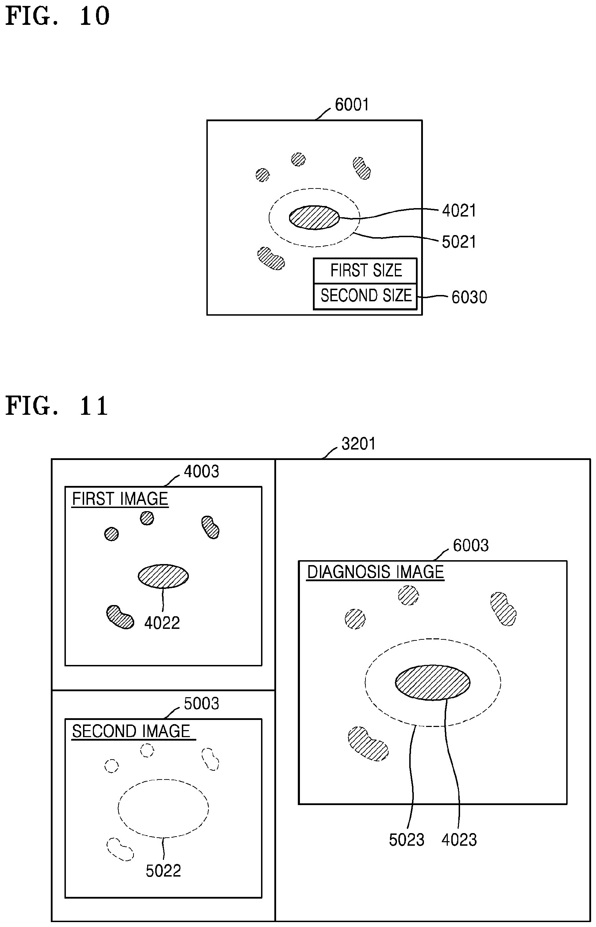

The display 3200 displays a screen image including a diagnosis image that shows the first information. The diagnosis image is acquired based on the registered image obtained by performing image registration with respect to the respective pieces of ultrasound data for the plurality of time points, and accordingly means an ultrasound image from which a user may visually recognize the first information. In detail, the diagnosis image may be an ultrasound image displayed so that states of the at least one target at the plurality of time points may be distinguished from one another. The diagnosis image that shows the first information will be described later in detail with reference to FIGS. 10 and 11.

An exemplary case where the respective pieces of ultrasound data for the plurality of time points acquired by the image processor 3100 include first ultrasound data acquired by scanning the object at a first time point and second ultrasound data acquired by scanning the object at a second time point will now be described. In other words, an exemplary case where the first information is acquired using pieces of ultrasound data respectively acquired by scanning the object at the first time point and the second time point, which is different from the first time point, will now be described.

In detail, the diagnosis image displayed on the display 3200 may be an ultrasound image in which a first target image of the at least one target based on the first ultrasound data and a second target image of the at least one target based on the second ultrasound data are overlappingly displayed.

FIG. 5B is a block diagram of an ultrasound image display apparatus 3050 according to another embodiment. The ultrasound image display apparatus 3050 of FIG. 5B may further include a communicator 3300 and a memory 3400, compared with the ultrasound image display apparatus 3000 of FIG. 5A. The components included in the ultrasound image display apparatus 3050 may be connected to one another via a bus 3500.

The communicator 3300 may receive respective pieces of ultrasound data for a plurality of time points from an external source. In detail, when the ultrasound image display apparatus 3050 does not acquire the respective pieces of ultrasound data for the plurality of time points via an ultrasound scan, the ultrasound image display apparatus 3050 may receive, from an external ultrasound diagnosis apparatus (not shown), respective pieces of ultrasound data for a plurality of time points acquired by scanning an object at different time points.

In detail, the communicator 3300 may receive first ultrasound data and second ultrasound data. The communicator 3300 may receive the first ultrasound data and the second ultrasound data simultaneously or at different times. The communicator 3300 may receive the first ultrasound data and the second ultrasound data from the ultrasound diagnosis apparatus 1000 or the server 32 of FIG. 1.

The memory 3400 may store at least one selected from the first ultrasound data and the second ultrasound data.

The first ultrasound data and the second ultrasound data may each refer to multidimensional data formed of discrete image elements (e.g., pixels in a two-dimensional (2D) image and voxels in a three-dimensional (3D) image). The first ultrasound data and the second ultrasound data may each be volume data formed of voxels. Each voxel may correspond to a voxel value, and the voxel value may be brightness and/or color information.

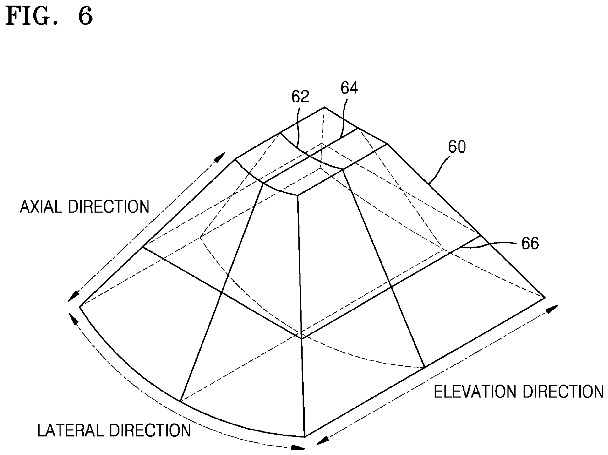

FIG. 6 illustrates an example of ultrasound data that is acquired by ultrasound image display apparatuses according to some embodiments. In FIG. 6, reference numerals 62, 64, and 66 represent a sagittal view, a coronal view, and an axial view, respectively, which intersect with one another. In FIG. 6, an axial direction indicates a direction in which an ultrasound signal travels with respect to a transducer of the ultrasound probe 20 of FIG. 1, a lateral direction indicates a direction in which a scan line moves, and an elevation direction is a depth direction of a 3D ultrasound image and indicates a direction in which a frame (i.e., a scanning plane) moves.

A case where an ultrasound image display apparatus according to an embodiment of the present invention is the ultrasound image display apparatus 3050 of FIG. 5B will now be described as an example.

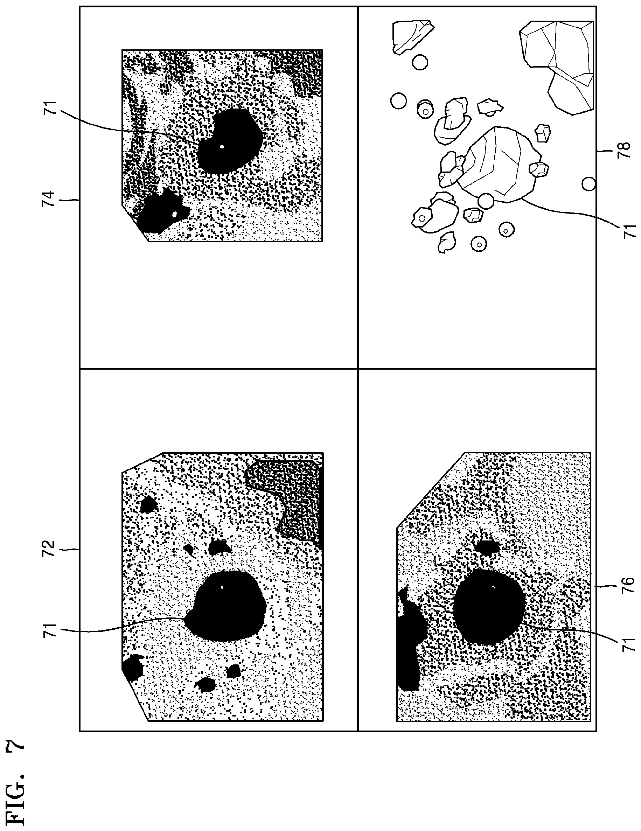

FIG. 7 illustrates ultrasound images acquired from such ultrasound data as FIG. 6.

Referring to FIG. 7, a plurality of ultrasound images 72, 74, 76, and 78 may be acquired from ultrasound data that is volume data. The ultrasound images 72, 74, and 76 may be cross-sectional images obtained by imaging a cross-section included in the volume data, and the ultrasound image 78 is a 3D ultrasound image obtained by volume-rendering the volume data. For example, the ultrasound images 72, 74, and 76 may represent the sagittal view 62, the coronal view 64, and the axial view 66 of FIG. 6, respectively.

The 3D ultrasound image 78 acquired from ultrasound data about an ovary shows a plurality of follicles or cysts having globular shapes. A follicle image 71 that is bulkiest among a plurality of follicle images each represented as a globular shape in the 3D ultrasound image 78 may be an image of a selected follicle, a polycystic ovary, in which ovulation is induced.

Circular dark areas in the ultrasound images 72, 74, and 76 may be images of follicles or cysts, because an area for a follicle or a cyst in the ultrasound data has low brightness. A follicle image 71 that is bulkiest in each of the ultrasound images 72, 74, and 76 may be a cross-sectional image of the selected follicle.

To diagnose whether the selected follicle grows normally, respective pieces of ultrasound data acquired by scanning an object at different time points may be used. However, since the object is scanned at the different time points, the position of the probe 20 of FIG. 1 scanning the object may vary. Accordingly, the respective pieces of ultrasound data acquired at the different time points are acquired in different coordinate systems, and thus the coordinate systems of the respective pieces of ultrasound data are different. There may exist an outlier that is present in one of the respective pieces of ultrasound data acquired at the different time points but is not present in the other pieces of ultrasound data. The size of a follicle may vary over time. These factors may make it difficult to diagnose whether the selected follicle grows normally by using ultrasound data. The ultrasound image display apparatuses 3000 and 3050 according to embodiments of the present invention overcome the difficulties in the diagnosis and thus acquire the first information by image-registering the respective pieces of ultrasound data for the plurality of time points and display the first information so that a user may easily ascertain a change in the target and easily diagnose the object.

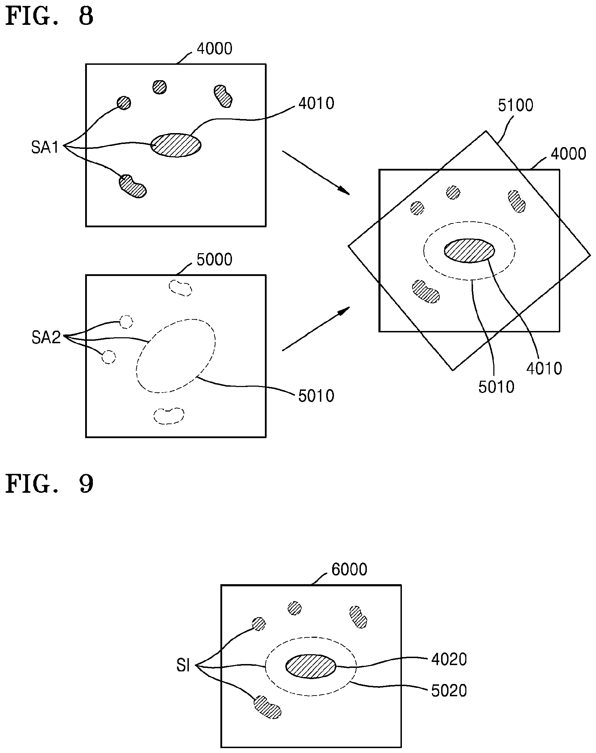

FIG. 8 illustrates image registration that is performed by image processors of ultrasound image display apparatuses according to some embodiments.

Referring to FIG. 8, first ultrasound data 4000 may include a plurality of first separate areas SA1, and second ultrasound data 5000 may include a plurality of second separate areas SA2. The first ultrasound data 4000 is acquired by scanning an object at a first time point, and the second ultrasound data 5000 is acquired by scanning the object at a second time point that is different from the first time point. Although FIG. 8 illustrates that the first ultrasound data 4000 and the second ultrasound data 5000 are 2D data, this is an example for convenience of explanation and illustration. The first ultrasound data 4000 and the second ultrasound data 5000 may each be volume data.

The first and second separate areas SA1 and SA2 may each be a group of voxels having voxel values that range within a predetermined range. When the ultrasound data 4000 and 5000 are data about an ovary, areas for follicles or cysts within the ultrasound data 4000 and 5000 have low brightness, and thus the voxels corresponding to the ultrasound data 4000 and 5000 may have low voxel values. The first and second separate areas SA1 and SA2 may each be a group of voxels having voxel values that are smaller than a threshold value. In other words, the first and second separate areas SA1 and SA2 may each be a group of voxels corresponding to a follicle or a cyst.

One of the first separate areas SA1 of the first ultrasound data 4000 may be a first target area 4010, and one of the second separate areas SA2 of the second ultrasound data 5000 may be a second target area 5010. Each of the first and second target areas 4010 and 5010 is a separate area of a target in which a change over time is to be monitored. In detail, the first target area 4010 represents a state of a predetermined target at the first time point, and the second target area 5010 represents a state of the predetermined target at the second time point. The target may be a selected follicle for which ovulation is induced from among the follicles included in the polycystic ovary 50 of FIG. 4B. The target that is to be monitored may be at least one follicle, but, for convenience of explanation, FIG. 8 and the drawings described below illustrate a case where the target is one follicle, in detail, one selected follicle.

Since the first ultrasound data 4000 and the second ultrasound data 5000 are acquired by scanning the object at different times, the first ultrasound data 4000 and the second ultrasound data 5000 are acquired in different coordinate systems. This is because, since the object is scanned at the different times, the position of the probe 20 of FIG. 1 scanning the object may vary.

The image processor 3100 of FIG. 5B performs image registration with respect to the first ultrasound data 4000 and the second ultrasound data 5000. The image registration is the process of transforming the first ultrasound data 4000 and the second ultrasound data 5000 into one coordinate system. The image processor 3100 may acquire second registered data 5100 by transforming the second ultrasound data 5000 so that the second ultrasound data 5000 is registered to the first ultrasound data 4000. On the other hand, the image processor 3100 may acquire first registered data (not shown) by transforming the first ultrasound data 4000 so that the first ultrasound data 4000 is registered to the second ultrasound data 5000. A case where the second ultrasound data 5000 is transformed to be registered to the first ultrasound data 4000 will now be described as an example. Image registration may be performed via various image processing techniques. For example, the image processor 3100 may acquire the second registered data 5100 by fixing the first ultrasound data 4000 and spatially registering the second ultrasound data 5000 to align with the first ultrasound data 4000. Alternatively, the image processor 3100 may acquire the second registered data 5100 by fixing the first ultrasound data 4000 and performing linear transformation, such as translation or rotation, on the second ultrasound data 5000.

When the first ultrasound data 4000 and the second ultrasound data 5000 are registered, at least one pair of separate areas SA1 and SA2 from among the first separate areas SA1 and the second separate areas SA2 may be registered. In particular, the first target area 4010 and the second target area 5010 may be registered. In other words, the first target area 4010 and the second target area 5010 may overlap with each other.

The first target area 4010 may be the bulkiest area from among the first separate areas SA1, and the second target area 5010 may also be the bulkiest area from among the second separate areas SA2. Alternatively, the target areas 4010 and 5010 may be a pair of separate areas SA1 and SA2 of which volume changes are the greatest from among pairs of the first and second separate areas SA1 and SA2 that have been registered.

FIG. 9 illustrates a diagnosis image 6000 that is acquired by image processors of ultrasound image display apparatuses according to some embodiments.

Referring to FIGS. 8 and 9, the diagnosis image 6000 is acquired based on the first ultrasound data 4000 and the second ultrasound data 5000 that have been registered. The diagnosis image 6000 may be a volume-rendered image obtained based on the first ultrasound data 4000 and the second registered data 5100. Alternatively, the diagnosis image 6000 may be a cross-sectional image acquired from the first ultrasound data 4000 and the second registered data 5100.

In detail, the diagnosis image 6000 represents first information that represents a change in at least one selected from the size, position, and number of the at least one target at the plurality of different time points.

In detail, the diagnosis image 6000 may include images SI of pairs of the registered separate areas SA1 and SA2. Each of the images SI may be an image of a pair of registered separate areas SA1 and SA2. The image processor may perform image processing so that the images SI are displayed distinguishably. Alternatively, the image processor may perform image processing so that the first separate area SA1 and the second separate area SA2 that are a pair of registered separate areas SA1 and SA2 may be displayed distinguishably. For example, the images SI of the pairs of the registered separate areas SA1 and SA2 in the diagnosis image 6000 may be distinguished from each other by an outline, a color, a pattern, or the like.

In particular, the diagnosis image 6000 may include a first target image 4020 and a second target image 5020. In the diagnosis image 6000, the first target image 4020 and the second target image 5020 may overlap with each other. The first target image 4020 is an image of the target that is based on the first ultrasound data 4000. In other words, the first target image 4020 may be an image of the target that is based on the voxel values of the first target area 4010. Similarly, the second target image 5020 is an image of the target that is based on the second ultrasound data 5000. In other words, the second target image 5020 may be an image of the target that is based on the voxel values of the second target area 5010.

Referring to FIG. 9, as described above, in the diagnosis image 6000, the first target image 4020 corresponding to a state of the target, which is a specific follicle included in an ovary, at the first time point and the second target image 5020 corresponding to a state of the target at the second time point are registered and overlapped. Accordingly, a user may easily recognize a change in the target between the first time point and the second time point from the diagnosis image 6000. Although a 2D diagnosis image is illustrated in FIG. 9 and the drawings described below, a 3D diagnosis image may be used.

The image processor may perform image processing so that the first target image 4020 and the second target image 5020 are displayed distinguishably in the diagnosis image 6000. For example, in the diagnosis image 6000, the first target image 4020 and the second target image 5020 may be distinguished from each other by different colors, different types of outlines, or different types of patterns.

Alternatively, the image processor may perform image processing so that a difference between the first target image 4020 and the second target image 5020 is emphasized in the diagnosis image 6000. For example, a portion of the diagnosis image 6000 that corresponds to the difference may be highlighted with a color that is distinguished from the colors of the other portions.

As such, the ultrasound image display apparatuses according to some embodiments make a user intuitively and easily recognize a change in the object over time. Thus, the user may easily diagnose the change in the object or the change in the target included in the object over time. When the target is a selected follicle for which ovulation is induced from among follicles included in a polycystic ovary, the ultrasound image display apparatuses according to some embodiments enable a user to easily recognize a changed in the size of the selected follicle over time and thus easily diagnose whether the selected follicle normally grows over time.

FIG. 10 illustrates a diagnosis image 6001 that is acquired by image processors of ultrasound image display apparatuses according to some embodiments.

Referring to FIGS. 8 and 10, the image processors may acquire a first size of the target based on the first ultrasound data 4000 and acquire a second size of the target based on the second ultrasound data 5000. In detail, the first size and the second size of the target may be respectively acquired based on the first target area 4010 and the second target area 5010. The first size may be at least one selected from the volume of the first target area 4010, the long-axis length thereof, the short-axis length thereof, the radius thereof, the diameter thereof, and the area of a cross-section thereof, and the second size may be at least one selected from the volume of the second target area 5010, the long-axis length thereof, the short-axis length thereof, the radius thereof, the diameter thereof, and the area of a cross-section thereof.

The display 3200 of FIG. 5B may display the diagnosis image 6001 in which the first target image 4021 and the second target image 5021 are overlappingly displayed, and may further display size information 6030 of the target. The size information 6030 of the target may include the first size and the second size. The size information 6030 may further include information about a change in the size of the target over time. For example, the information about the change in the size of the target over time may be a difference between the first size and the second size or a size change rate based on the first size and the second size.

FIG. 11 illustrates a screen 3201 of a display of an ultrasound image display apparatus according to an embodiment.

Referring to FIGS. 8 and 11, a first image 4003 and a second image 5003 may be displayed together with a diagnosis image 6003 on the screen 3201 of the display. In the diagnosis image 6003, a first target image 4023 and a second target image 5023 overlap with each other. The first image 4003 and the second image 5003 are acquired based on respective pieces of registered ultrasound data for a plurality of time points, in detail, based on the first ultrasound data 4000 and the second registered data 5100, and are respective ultrasound images for a plurality of time points that are displayed in an identical coordinate system. In detail, the first image 4003 includes a first target image 4022 as an image based on the first ultrasound data 4000, and the second image 5003 includes a second target image 5022 as an image based on the second registered data 5100. The first image 4003 may be obtained by volume-rendering the first ultrasound data 4000, and the second image 5003 may be obtained by volume-rendering the second registered data 5100. Alternatively, the first image 4003 may be a cross-sectional image including a cross-section of the first target area 4010 in the first ultrasound data 4000, and the second image 5003 may be a cross-sectional image including a cross-section of the second target area 5010 in the second registered data 5100. Each of the respective cross-sections of the first target area 4010 and the second target area 5010 in the second registered data 5100 may be a cross-section of an image obtained by registering the first ultrasound data 4000 and the second ultrasound data 5000.

As illustrated in FIG. 11, the first image 4003 and the second image 5003 may be displayed simultaneously on the screen 3201. Alternatively, the first image 4003 and the second image 5003 may be sequentially displayed on the screen 3201. When the first ultrasound data 4000 is acquired by scanning the object at a first time point and the second ultrasound data 5000 is acquired by scanning the object at a second time point subsequent to the first time point, the first image 4003 may be first displayed and the second image 5003 may be then displayed.

As such, the ultrasound image display apparatuses according to some embodiments make a user intuitively and easily recognize a change in the object over time, by displaying a diagnosis image acquired by registering the first and second ultrasound data.

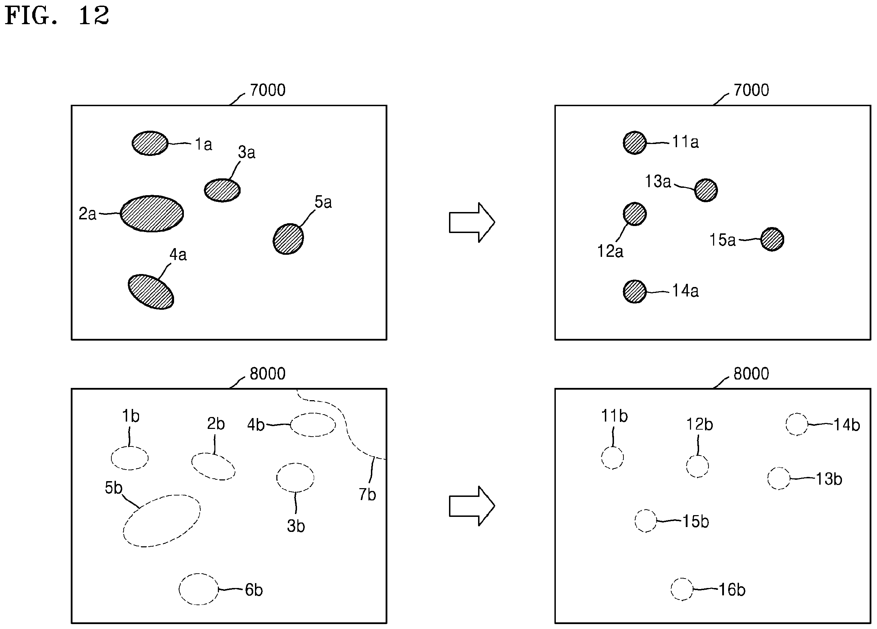

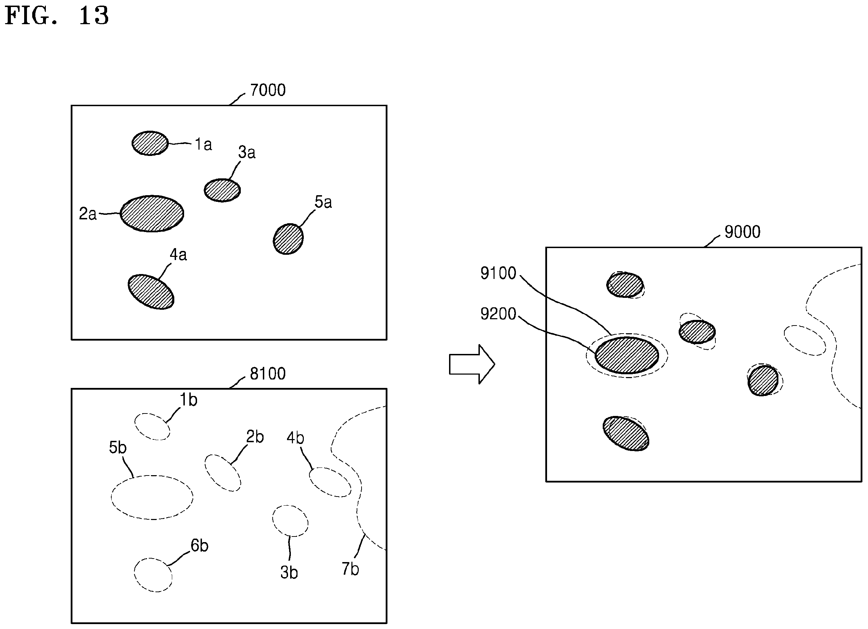

FIGS. 12 and 13 illustrate processes in which an image processor of an ultrasound image display apparatus according to an embodiment acquires a diagnosis image via image registration.

Referring to FIG. 12, the image processor 3100 of FIG. 5B may respectively detect a plurality of separate areas 1a-5a and a plurality of separate areas 1b-7b by respectively segmenting the first ultrasound data 7000 and the second ultrasound data 8000. Although FIG. 12 illustrates that the first ultrasound data 7000 and the second ultrasound data 8000 are 2D data, this is an example for convenience of explanation and illustration. The first ultrasound data 7000 and the second ultrasound data 8000 may each be volume data.

The first ultrasound data 7000 may include the plurality of separate areas 1a-5a, and the second ultrasound data 8000 may include the plurality of separate areas 1b-7b. Each of the separate areas 1a-5a and 1b-7b may be a group of pixels or voxels corresponding to a follicle or a cyst. The image processor may segment the first ultrasound data 7000 and the second ultrasound data 8000, based on the pixel values of the pixels or the voxel values of the voxels. Each of the separate areas 1a-5a and 1b-7b may be an area formed of a group of voxels having voxel values that range within a predetermined range. The image processor may label the separate areas 1a-5a in the first ultrasound data 7000 and the separate areas 1b-7b in the second ultrasound data 8000 so that the separate areas 1a-5a are distinguished from the separate areas 1b-7b.

The image processor 3100 may perform image registration with respect to the first ultrasound data and the second ultrasound data via a random sample consensus (RANSAC). The RANSAC is a method of randomly selecting pieces of sample data and then selecting pieces of sample data that reach a maximum consensus from among the randomly selected pieces of sample data. An outlier may be removed via the RANSAC. The outlier may be present in the first ultrasound data but may be absent in the second ultrasound data, or vice versa. In FIG. 12, the separate area 7b of the second ultrasound data 8000 corresponds to the outlier. The outlier may reduce the accuracy of image registration. Accordingly, the accuracy of image registration may be increased by removing the outlier via the RANSAC.

The image processor may detect reference points 11a-15a for the separate areas 1a-5a included in the first ultrasound data 7000 and reference points 11b-16b for the separate areas 1b-6b except for the outlier included in the second ultrasound data 8000. The reference points 11a-15a and 11b-16b may be centroid points or average points of the separate areas 1a-5a and 1 b-6b, respectively.

The image processor may acquire volume information for each of the separate areas 1a-5a and 1b-6b. For example, the volume information may include at least one of the volume, long-axis length, short-axis length, shape, and the like of each of the separate areas 1a-5a and 1b-6b.

Referring to FIGS. 12 and 13, the image processor may register the first ultrasound data 7000 and the second ultrasound data 8000 to thereby acquire second registered data 8100 in which the second ultrasound data 8000 is transformed to be registered to the first ultrasound data 7000. The second registered data 8100 may be obtained based on matching between the first reference points 11a-15a included in the first ultrasound data 7000 and the second reference points 11b-16b included in the second ultrasound data 8000.

The image processor may acquire a diagnosis image 9000 based on the first ultrasound data 7000 and the second registered data 8100. In the diagnosis image 9000, a first target image 9100 and a second target image 9200 may overlap with each other. Since the above descriptions of a diagnosis image are all applicable to the diagnosis image 9000, redundant descriptions thereof will be omitted.

The image processor may respectively detect target areas 2a and 5b which are separate areas of a target, from the plurality of separate areas 1a-5a of the first ultrasound data 7000 and the plurality of separate areas 1b-7b of the second ultrasound data 8000. The respective target areas 2a and 5b of the first ultrasound data 7000 and the second ultrasound data 8000 may correspond to the first and second target areas 4010 and 5010 of FIG. 8, respectively. Accordingly, since the above descriptions of the first and second target areas 4010 and 5010 are all applicable to the target areas 2a and 5b, redundant descriptions thereof will be omitted.

The target areas 2a and 5b may be detected based on the volume information about each of the separate areas 1a-5a and 1b-6b. For example, the target areas 2a and 5b may be detected based on the shapes of the separate areas 1a-5a and 1b-6b and the volumes of the separate areas 1a-5a and 1b-6b. Alternatively, the target areas 2a and 5b may be detected after image registration is completed.

The image processor may perform image registration with respect to the first ultrasound data 7000 and the second ultrasound data 8000 by using an iterative closest point (ICP).

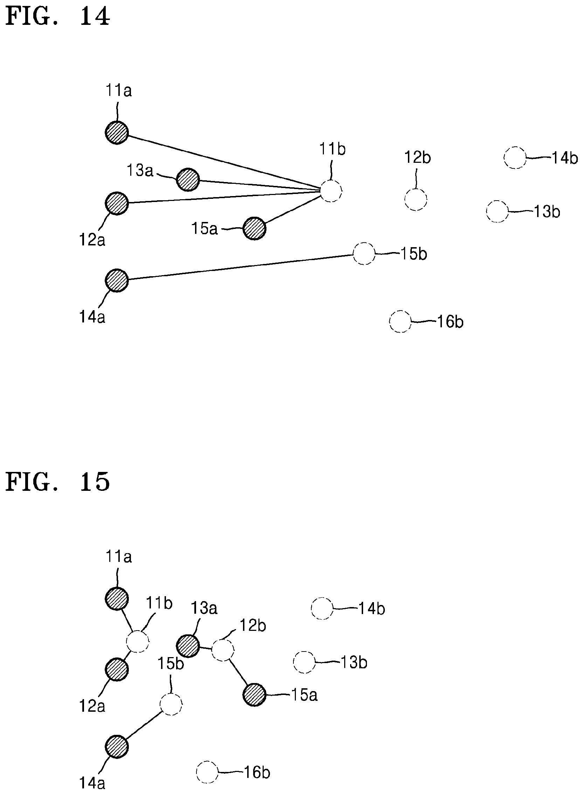

FIGS. 14-17 illustrate image registration that is performed using an ICP.

Referring to FIG. 14, the image processor may detect a reference point that is closest to each of the reference points 11a-15a of the first ultrasound data 7000 from among the reference points 11b-16b of the second ultrasound data 8000 and match the detected closest reference points with the reference points 11a-15a.

The reference points 11a, 12a, 13a, and 15a of the first ultrasound data 7000 may be matched with the reference point 11b that is closest thereto from among the reference points 11b-16b of the second ultrasound data 8000, and the reference point 14a of the first ultrasound data 7000 may be matched with the reference point 15b that is closest thereto from among the reference points 11b-16b of the second ultrasound data 8000.

When matching each of the reference points 11a-15a of the first ultrasound data 7000 with one of the reference points 11b-16b of the second ultrasound data 8000, the image processor may perform the matching based on a distance between the reference points and volume information between the reference points. The image processor may match the reference points by applying a weight to each of the reference points based on the volume information. For example, a higher weight may be applied to a reference point of the second ultrasound data having similar volume information to the volume information of a reference point of the first ultrasound data than to the other reference points. On the other hand, a lower weight may be applied to a reference point of the second ultrasound data having volume information not similar to the volume information of a reference point of the first ultrasound data than to the other reference points. In other words, different weights may be applied to the plurality of reference points. The weights that are respectively applied to the reference points may be determined based on pieces of volume information about the separate areas corresponding to the reference points.

The image processor may transform the second ultrasound data based on a result of the matching. For example, the image processor may acquire a translation degree and/or a rotation degree of the second ultrasound data, based on a result of the matching, and accordingly may perform linear transformation on the second ultrasound data.

FIG. 15 illustrates the reference points 11a-15a of the first ultrasound data 7000 and reference points 11b-16b of the second ultrasound data 8000 that have been obtained via transformation according to a result of the matching illustrated in FIG. 14.

Referring to FIG. 15, the image processor may match a reference point that is closest to each of the reference points 11a-15a of the first ultrasound data 7000 from among the reference points 11b-16b of the second ultrasound data 8000 with each of the reference points 11a-15a.

The reference points 11a and 12a of the first ultrasound data 7000 may be matched with the reference point 11b of the second ultrasound data 8000, the reference points 13a and 15a of the first ultrasound data 7000 may be matched with the reference point 12b of the second ultrasound data 8000, and the reference point 14a of the first ultrasound data 7000 may be matched with the reference point 15b of the second ultrasound data 8000.

The image processor may transform again the second ultrasound data 800 based on a result of the matching.

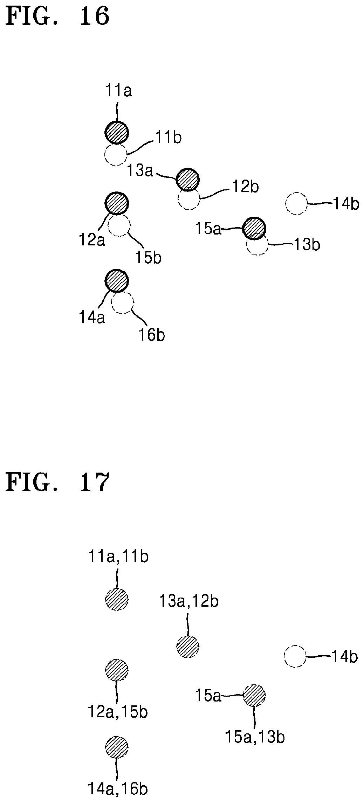

FIG. 16 illustrates the reference points 11a-15a of the first ultrasound data 7000 and reference points 11b-16b of the second ultrasound data 8000 that have been obtained via transformation according to a result of the matching illustrated in FIG. 15.

Referring to FIG. 16, the image processor may match again a reference point that is closest to each of the reference points 11a-15a of the first ultrasound data 7000 from among the reference points 11b-16b of the second ultrasound data 8000 with each of the reference points 11a-15a. The reference points 11a, 12a, 13a, 14a, and 15a of the first ultrasound data 7000 may be matched with the reference points 11b, 15b, 12b, 16b, and 13b of the second ultrasound data 8000, respectively. The image processor may transform again the second ultrasound data according to a result of the matching.

FIG. 17 illustrates the reference points 11a-15a of the first ultrasound data 7000 and reference points 11b-16b of the second ultrasound data 8000 that have been obtained via transformation according to a result of the matching illustrated in FIG. 16.

Referring to FIG. 17, each of the reference points 11a-15a of the first ultrasound data 7000 coincides with one of the reference points 11b-16b of the second ultrasound data 8000. Accordingly, image registration of the first ultrasound data and the second ultrasound data is completed.

Referring back to FIG. 13, after the image registration is completed, the image processor may respectively detect the target areas 2a and 5b from the plurality of separate areas 1a-5a of the first ultrasound data 7000 and the plurality of separate areas 1b-7b of the second registered data 8100. The target areas 2a and 5b may be detected based on the volume information about each of the separate areas 1a-5a and 1b-6b. For example, the target areas 2a and 5b may be detected based on at least one selected from the shapes of the separate areas 1a-5a and 1b-6b, the volumes thereof, and volume variations thereof.

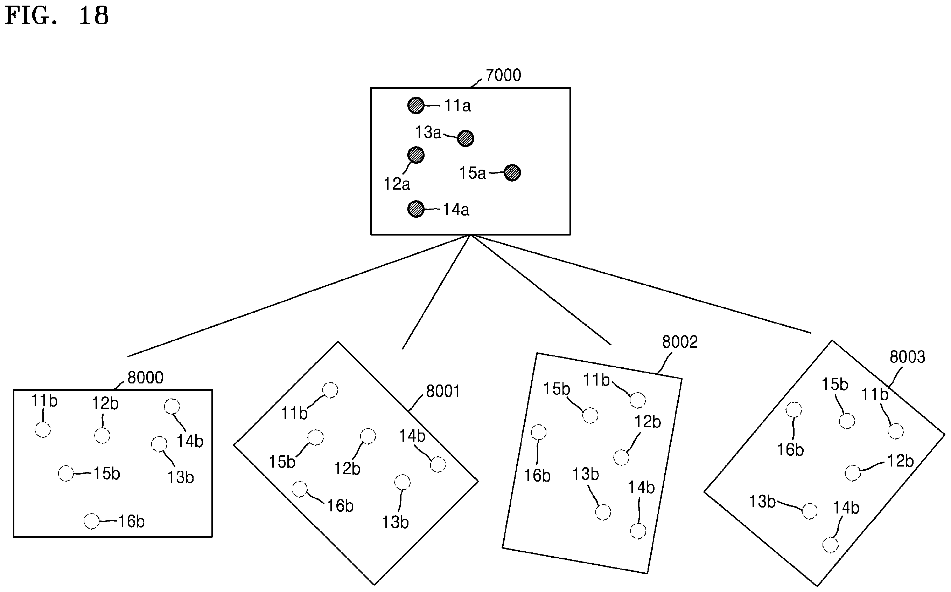

FIG. 18 illustrates ultrasound data processing for image registration that is performed by an image processor of an ultrasound image display apparatus according to an embodiment.

Referring to FIG. 18, to register the first ultrasound data 7000 and the second ultrasound data 8000, the image processor may acquire second ultrasound data sets 8000, 8001, 8002, and 8003 by transforming the second ultrasound data 8000 variously. The second ultrasound data sets 8000, 8001, 8002, and 8003 may be acquired by spatially transforming or linearly transforming the second ultrasound data 8000. The image processor may perform image registration with respect to the first ultrasound data 7000 and transformed second ultrasound data that is included in each of the second ultrasound data sets 8000, 8001, 8002, and 8003, by using the ICP.

When the first ultrasound data 7000 and the second ultrasound data 8000 are greatly misaligned, an error may occur during reference point matching via the ICP, and thus the accuracy of image registration may be reduced. Accordingly, when the second ultrasound data 8000 is variously transformed and then registered with the first ultrasound data 7000, the accuracy of image registration may be increased.

As such, the image processor may perform image registration with respect to the first ultrasound data and the second ultrasound data via the ICP. The image processor may perform image registration with respect to the first ultrasound data and the second ultrasound data via various image registration methods other than the ICP. For example, the image registration may be performed using mutual information, a correlation coefficient, ratio-image uniformity, or partitioned intensity uniformity.

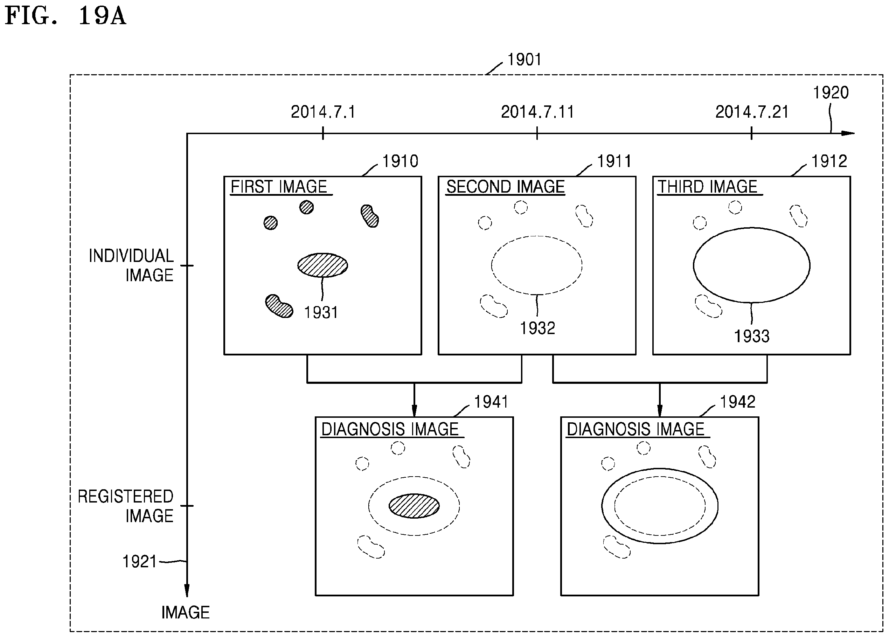

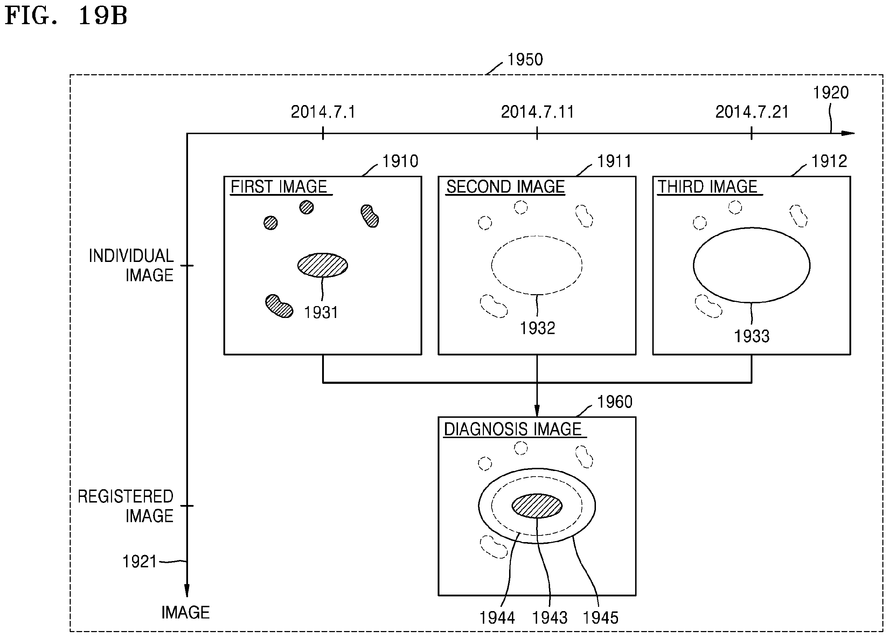

FIGS. 19A and 19B illustrate screen images that are displayed by an ultrasound image display apparatus according to an embodiment. In detail, FIG. 19A illustrates a screen image 1901 that is displayed on the display 3200 of FIG. 5B. FIG. 19B illustrates a screen image 1950 that is displayed on the display 3200 of FIG. 5B.

The image processor 3100 may generate respective ultrasound images for a plurality of time points, based on respective pieces of ultrasound data for a plurality of time points. FIGS. 19A and 19B illustrate a case of using ultrasound data acquired by scanning an object at three different time points which are a first time point, a second time point, and a third time point.

In detail, referring to FIG. 19A, the image processor 3100 acquires a first image 1910 by using first ultrasound data acquired by scanning the object at the first time point, acquires a second image 1911 by using second ultrasound data acquired by scanning the object at the second time point, and acquires a third image 1912 by using third ultrasound data acquired by scanning the object at the third time point. The image processor 3100 may acquire at least one diagnosis image, namely, diagnosis images 1941 and 1942, including first information, by performing image registration with respect to the first ultrasound data, the second ultrasound data, and the third ultrasound data.

The screen image 1901 displayed on the display 3200 may include the ultrasound images 1910, 1911, and 1912 respectively acquired based on the respective pieces of ultrasound data for the plurality of time points. The respective ultrasound images 1910, 1911, and 1912 may be arranged in the ascending or descending order of the plurality of time points at which the object was scanned. In detail, the time points may be arranged on an axis 1920 of the screen image 1901, and images may be arranged on another axis 1921 thereof.

Referring to FIG. 19A, the first image 1910 acquired by scanning the object on July 1, 2014, which is the first time point, includes a target area 1931 representing a target. The second image 1911 acquired by scanning the object on July 11, 2014, which is the second time point, includes a target area 1932 representing a target. The third image 1912 acquired by scanning the object on July 21, 2014, which is the third time point, includes a target area 1933 representing a target. As illustrated in FIG. 19A, the first image 1910, the second image 1911, and the third image 1912 may be arranged on a first row of the screen image 1901. The diagnosis image 1941 acquired by registering the first image 1910 and the second image 1911 and the diagnosis image 1942 acquired by registering the second image 1911 and the third image 1912 may be arranged on a second row of the screen image 1901.

A user may easily ascertain a change in the target between two different time points from the screen image 1901.

A description of FIG. 19B that is the same as given above with reference to FIG. 19A will not be repeated herein.

Referring to FIG. 19B, the image processor 3100 may generate a diagnosis image 1960 in which a change in states of the target at the first time point, the second time point, and the third time point is displayed.

In detail, the image processor 3100 may perform image registration with respect to the first image 1910, the second image 1911, and the third image 1912 and acquire the diagnosis image 1960 in which the first image 1910, the second image 1911, and the third image 1912 that have been registered are overlapped with one another and displayed.

Accordingly, in the diagnosis image 1960 included in the screen image 1950, a first target area 1943 corresponding to a first target area 1931 representing a target at the first time point, a second target area 1944 corresponding to a second target area 1932 representing a target at the second time point, and a third target area 1945 corresponding to a third target area 1933 representing a target at the third time point are overlapped with one another and displayed. A user may easily ascertain a change in the target over time from the screen image 1950.

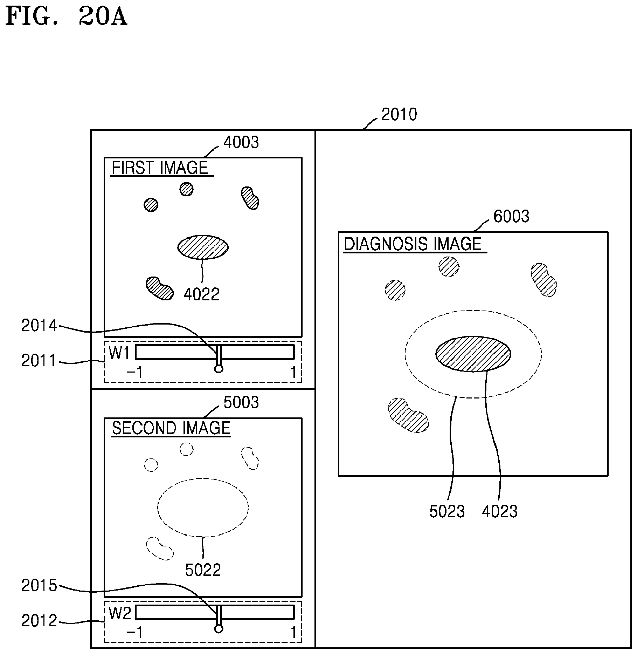

FIG. 20A illustrates a screen image that is displayed by an ultrasound image display apparatus according to an embodiment. In detail, FIG. 20A illustrates a screen image that is displayed on the display 3200.

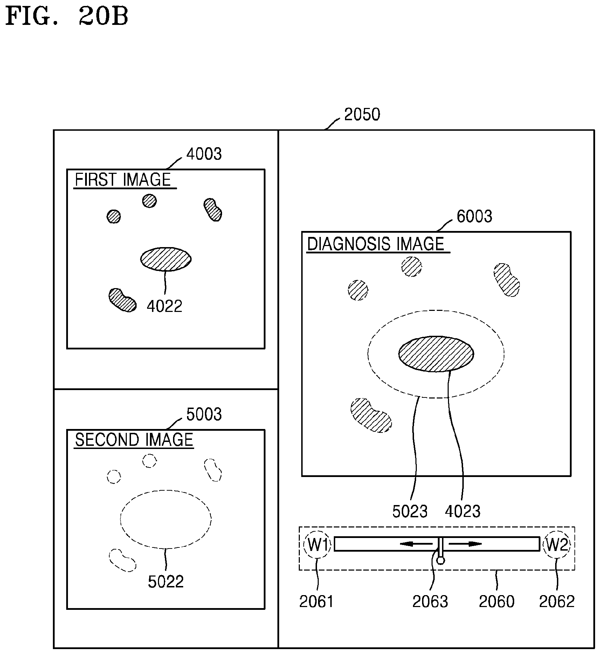

The image processor 3100 acquires respective ultrasound images for a plurality of time points based on respective pieces of registered ultrasound data for the plurality of time points and sets a weight for each of the respective ultrasound images for the plurality of time points. The weight is a value that is applied to an ultrasound image for a corresponding time point so that the ultrasound image for the corresponding time point is more distinctly displayed or less distinctly displayed on a diagnosis image. The weight may be set by a user or by the image processor 3100. A diagnosis image 6003 is an image in which respective ultrasound images for the plurality of time points weighted by applying the respective weights to the respective ultrasound images for the plurality of time points are overlapped with one another and displayed. FIGS. 20A and 20B illustrate a case where a user sets a weight.

In detail, FIG. 20A illustrates a user interface (UI) image 2010 for individually setting weights that are to be applied to a first image 4003 and a second image 5003. FIG. 20B illustrates a UI image 2050 for simultaneously setting the weights that are to be applied to the first image 4003 and the second image 5003.

Referring to FIG. 20A, the UI image 2010 may include a first menu 2011 for setting a first weight that is applied to the first image 4003 and a second menu 2012 for setting a second weight that is applied to the second image 5003.

The first menu 2011 may include a cursor 2014 setting a weight within a settable weight range (e.g., from -1 to 1), and the second menu 2012 may include a cursor 2015 setting a weight within a settable weight range (e.g., from -1 to 1). When a weight is set to be a lower limit (for example, -1), an image to which the weight is to be applied is displayed with the lightest brightness within the diagnosis image 6003. When a weight is set to be an upper limit (for example, 1), an image to which the weight is to be applied is displayed with the deepest brightness within the diagnosis image 6003. When a weight is set to be an intermediate value within the settable weight range, an image to which the weight is to be applied is displayed with the same brightness as the brightness of the non-weighted image within the diagnosis image 6003.

In detail, the first weight applied to the first image 4003 is 0 and the second weight applied to the second image 5003 is 0, the diagnosis image 6003 may be displayed the same as the diagnosis image 6001 of FIG. 10 and the diagnosis image 6003 of FIG. 11. When the first weight applied to the first image 4003 is -1 and the second weight applied to the second image 5003 is 1, the first target image 4023 may be displayed with a lighter color and the second target image 5023 may be displayed with a deep color, within the diagnosis image 6003. When the first weight applied to the first image 4003 is 1 and the second weight applied to the second image 5003 is 1, both the first target image 4023 and the second target image 5023 may be displayed with the deepest color in the diagnosis image 6003. When the first weight applied to the first image 4003 is -1 and the second weight applied to the second image 5003 is -1, both the first target image 4023 and the second target image 5023 may be displayed with the lightest color in the diagnosis image 6003.

Referring to FIG. 20B, the UI image 2050 may include a third menu 2060 for setting, all at one time, the weights that are applied to the first image 4003 and the second image 5003.

The third menu 2060 may include a cursor 2063 for setting a weight.

For example, in the third menu 2060, when the cursor 2063 is moved toward a weight W1 that is applied to the first image 4003, the weight of the first image 4003 increases, and a second weight W2 that is applied to the second image 5003 decreases. Then, in the diagnosis image 6003, the first target image 4023 may be displayed with a deeper brightness than the second target image 5023, and the second target image 5023 may be displayed with a lighter color than the first target image 4023.

As another example, in the third menu 2060, when the cursor 2063 is positioned at 0, which is a middle between the first and second weights W1 and W2, the first target image 4023 and the second target image 5023 may be displayed to the same degree of brightness, and thus the diagnosis image 6003 may be displayed the same as the diagnosis images 6001 and 6003 of FIGS. 10 and 11.

As another example, in the third menu 2060, when the cursor 2063 is moved toward a weight W2 that is applied to the second image 5003, the weight of the second image 5003 increases, and the first weight W1 applied to the first image 4003 decreases. Then, in the diagnosis image 6003, the first target image 4023 may be displayed with a lighter color than the second target image 5023, and the second target image 5023 may be displayed with a deeper color than the first target image 4023.

As described above, a target image at a specific time point may be displayed more clearly than target images at the other time points according to user's intentions by using the weight setting. Thus, a diagnosis image that conforms to a user intention may be output.

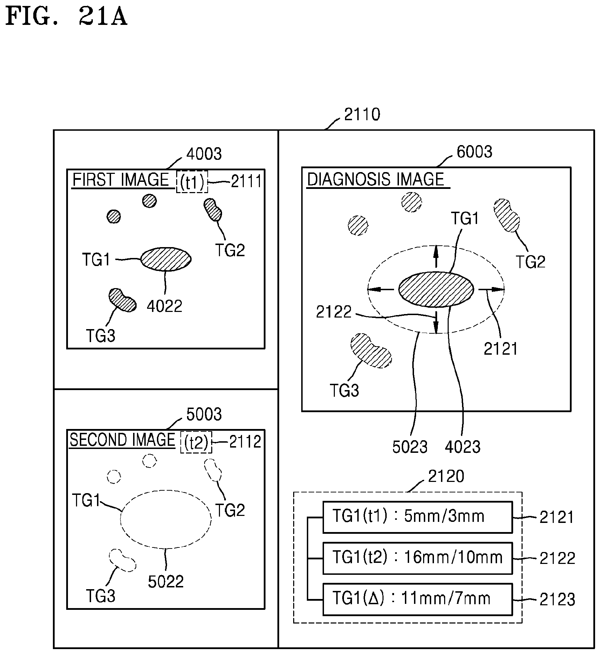

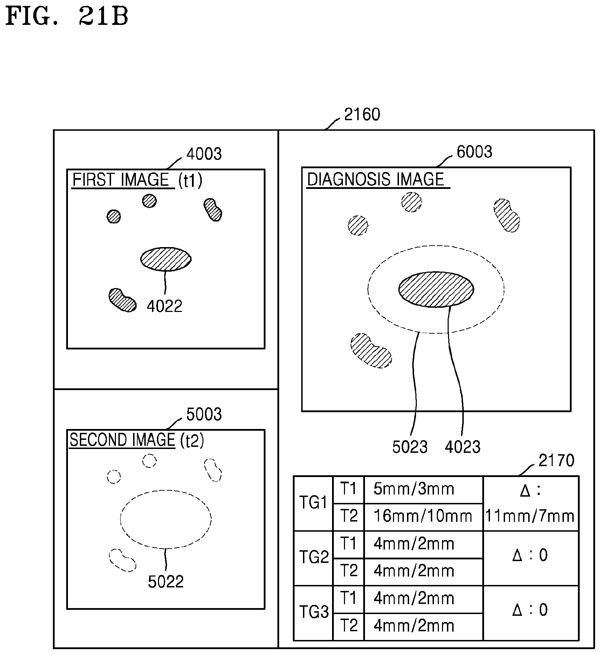

FIGS. 21A and 21B illustrate other screen images that are displayed by an ultrasound image display apparatus according to an embodiment.

Referring to FIG. 21A, a screen image 2110 displayed on the display 3200 may further include target change numeric information that numerically represents a change in at least one selected from the size, position, and number of at least one target. In detail, the target change numeric information may include the value of at least one selected from an area, a volume, a long-axis length, a short-axis length, a radius, a diameter, and a circumference that represent the size of the at least one target. The target change numeric information described with reference to FIGS. 21A and 21B may be a more detailed version of the size information 6030 of FIG. 10.

In detail, FIG. 21A illustrates the screen image 2110 on which target change numeric information about a target whose state has changed between a plurality of time points, which is included in an object, is displayed. FIG. 21B illustrates a screen image 2160 on which target change numeric information about all separate targets included in the object is displayed.

An identification indicator (for example, TG1, TG2, or TG3) for identifying a target that is correlated with at least one target is displayed on the first image 4003, the second image 5003, and the diagnosis image 6003 included in the screen image 2110. In detail, as illustrated in the screen image 2110, the identification indicator (for example, TG1) indicating a target may be labelled to an identical target (for example, 4022, 5022, 4023, and 5023) included in the first image 4003, the second image 5003, and the diagnosis image 6003.

In the first image 4003 and the second image 5003 included in the screen image 2110 displayed on the display 3200, information 2111 and information 2112 indicating the time points at which the first image 4003 and the second image 5003 are respectively acquired may be displayed.

Referring to FIG. 21A, the screen image 2110 may include target change numeric information 2120. The target change numeric information 2120 may display only target change numeric information about a state-changed target, for example, a selected follicle, and may not display information about a state-unchanged target. In detail, during the first time point t1 corresponding to the first image 4003 and the second time point t2 corresponding to the second image 5003, when a state change occurs only between the selected follicles 4022 and 5022 and the states of the other follicles are not changed, the target change numeric information 2120 may only display information about the target TG1 which is the state-changed target. The target change numeric information 2120 may include a variation 2123 (for example, TG1(Δ)) of the value of at least one selected from an area, a volume, a long-axis length, a short-axis length, a radius, a diameter, and a circumference that represent the size of the at least one target (for example, TG1).

In detail, FIG. 21A illustrates a case where the target change numeric information 2120 includes long-axis and short-axis lengths 2121 of the target 4022 (TG1) at the first time point t1, long-axis and short-axis lengths 2122 of the target 5022 (TG1) at the second time point t2, and the variation 2123 (for example, TG1(Δ)) between the first time point t1 and the second time point t2.

FIG. 21B illustrates a screen image 2160 on which target change numeric information about all separate targets included in the object is displayed.

Referring to C FIG. 21B, the screen image 2160 may include target change numeric information 2170 about the all separate targets included in the object. In detail, as illustrated in FIG. 21B, the target change numeric information 2170 may include information that represents sizes that the all separate targets (for example, TG1, TG2, and TG3) included in the ultrasound-scanned object have at the first time point t1 and the second time point t2.

In detail, in the target change numeric information 2170, size information about each of the separate targets included in the object may be displayed in the form of a list.

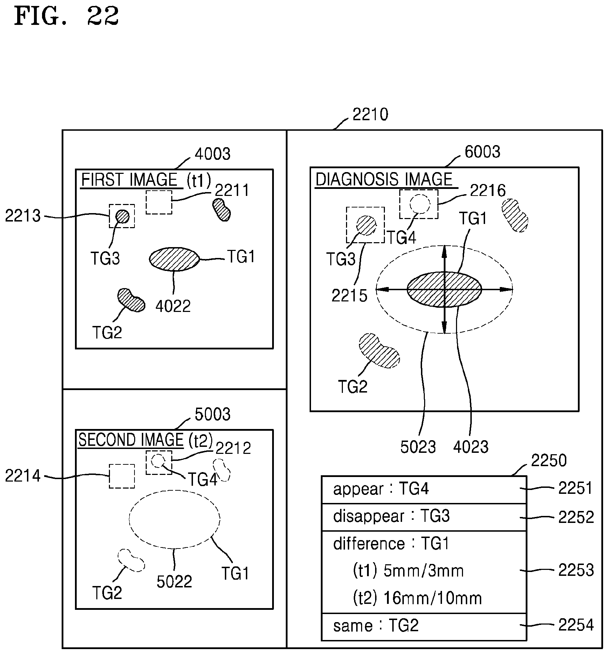

FIG. 22 illustrates a screen image 2210 that is displayed by an ultrasound image display apparatus according to an embodiment. A repeated description of the screen image 2110 given above with reference to FIG. 21A is omitted in the description of the screen image 2210 of FIG. 22.

The image processor 3100 of FIG. 5B may generate state change information 2250 representing state changes of all independent targets included in an object , based on respective pieces of ultrasound data for a plurality of time points. The display 3200 of FIG. 5B may display the screen image 2210 including the generated state change information 2250.

In detail, referring to FIG. 22, the screen image 2210 may include state change information 2250 representing state changes of independent targets (e.g., follicles) included in the object between the first and second time points t1 and t2. In detail, the state change information 2250 may include information 2251 about a target newly produced between the first and second time points t1 and t2, information 2252 about a target having disappeared between the first and second time points t1 and t2, information 2253 about a target having changed between the first and second time points t1 and t2, and information 2254 about a target having unchanged between the first and second time points t1 and t2.

Referring to FIG. 22, the independent targets TG1, TG2, and TG3 are included in the first image 4003 at the first time point t1, the target TG3 positioned at a location 2213 on the first image 4003 has disappeared at the second time point t2, and a target TG4 has appeared at a location 2212 on the second image 5003. During the first time point t1 and the second time point t2, the target TG2 has not changed, and the target TG1 has changed. The state change information 2250 includes information representing a change between targets included in the object during the first time point t1 and the second time point t2.

A user may easily ascertain a change in the object between the plurality of different time points from the state change information 2250.

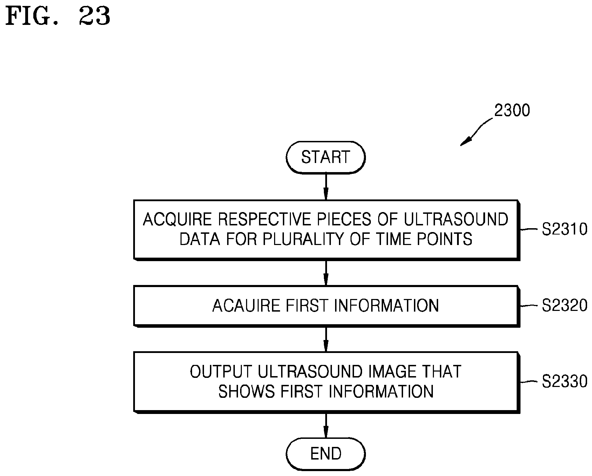

FIG. 23 is a flowchart of an ultrasound image displaying method 2300 according to an embodiment. The ultrasound image displaying method 2300 may be performed by the ultrasound image display apparatuses 3000 and 3050 according to the embodiments of the present invention described above with reference to FIGS. 1-22. Operations included in the ultrasound image displaying method 2300 are the same as the operations of the ultrasound image display apparatuses 3000 and 3050, and the technical spirit of the ultrasound image displaying method 2300 is the same as that of the ultrasound image display apparatuses 3000 and 3050. Accordingly, descriptions of the ultrasound image displaying method 2300 that are the same as given with reference to FIGS. 1-22 are not repeated herein.

Referring to FIG. 23, in operation S2310, respective pieces of ultrasound data for a plurality of time points, which represent an object including at least one target at a plurality of different time points are acquired. In detail, in operation S2310, the respective pieces of ultrasound data for the plurality of time points are acquired by scanning the object including at least one target at the plurality of different time points. The operation 2310 may be performed by the image processor 3100. An exemplary case where the respective pieces of ultrasound data for the plurality of time points acquired by the image processor 3100 include first ultrasound data acquired by scanning the object at a first time point, and second ultrasound data acquired by scanning the object at a second time point described in FIG. 8.

In operation S2320, first information representing a change in the at least one target at the plurality of different time points is acquired based on a correspondence between the acquired respective pieces of ultrasound data for the plurality of time points. In detail, first information that represents a change in at least one selected from the size, position, and number of the at least one target at the plurality of different time points is acquired by performing image registration with respect to the respective pieces of ultrasound data for the plurality of time points. The operation 2320 may be performed by the image processor 3100.

In operation S2330, a screen image including a diagnosis image that shows the first information is displayed. The operation S2330 may be performed by the display 3200.

Referring back to FIG. 1, the probe 20 may scan the object at different times. The object may include a polycystic ovary. The ultrasound transceiver 100 may acquire the first ultrasound data and the second ultrasound data by processing echo signals respectively received from the probe 20 at the different times.

The first ultrasound data is acquired by scanning the object at the first time point, and the second ultrasound data is acquired by scanning the object at the second time point. The second time point may be several days after the first time point.

Referring back to FIGS. 1 and 5B, when the ultrasound image display apparatus 3050 is included in the ultrasound diagnosis apparatus 1000 of FIG. 1, the first ultrasound data and the second ultrasound data may be acquired by scanning the object at different times by using the probe 20 of FIG. 1. The ultrasound image display apparatus 3050 may store at least one selected from the first ultrasound data and the second ultrasound data in the memory 3400.

When the ultrasound image display apparatus 3000 is the medical apparatus 34 or the portable terminal 36 connected to the ultrasound diagnosis apparatus 1000 of FIG. 1 via the network 30, the communicator 3300 of the ultrasound image display apparatus 3050 may receive the first ultrasound data and the second ultrasound data from the ultrasound diagnosis apparatus 1000 of FIG. 1. The communicator 3300 may receive the first ultrasound data and the second ultrasound data simultaneously or at different times. The memory 3400 may store at least one selected from the first ultrasound data and the second ultrasound data.

As described above, in an ultrasound image display apparatus and an ultrasound image displaying method according to an exemplary embodiment of the present inventive concept, when an object needs to be observed at an interval of time, a user may easily observe changes in the object at subsequent time points. In detail, in an ultrasound image display apparatus and an ultrasound image displaying method according to an exemplary embodiment of the present inventive concept, when a target included in an object needs to be monitored at a plurality of time points in order to diagnose or cure a gynecological disease, such as a myoma included in at least one follicle included in an ovary or a myoma of the uterus, a user may easily visually recognize a change in the object. The above-described exemplary embodiments can be written as computer programs and can be implemented in general-use digital computers that execute the programs using a computer readable recording medium.

Examples of the computer readable recording medium include magnetic storage media (e.g., ROM, floppy disks, hard disks, etc.), optical recording media (e.g., CD-ROMs, or DVDs), etc.

## Claims

1. An ultrasound image display apparatus (3000) comprising:
an image processor (3100) which acquires respective pieces of ultrasound data for a plurality of time points, which represent an object including at least one target at a plurality of different time points, and acquires first information representing a change in the at least one target at the plurality of different time points, based on a correspondence between the respective pieces of ultrasound data for the plurality of time points; and
a display (3200) which displays a screen image including a diagnosis image that shows the first information,
wherein the image processor (3100) acquires respective ultrasound images for a plurality of time points based on the respective pieces of ultrasound data for the plurality of time points and sets a weight for each of the respective ultrasound images for the plurality of time points, and generates the diagnosis image that is an image in which respective ultrasound images for the plurality of time points for each of which the weight is set are overlapped with one another and displayed, and
wherein the weight is a value that is applied to each of the respective ultrasound images for the plurality of time points so that each of the respective ultrasound images for the plurality of time points is more distinctly displayed or less distinctly displayed on the diagnosis image.

2. The ultrasound image display apparatus (3000) of claim 1, wherein the diagnosis image is an ultrasound image displayed so that states of the at least one target at the plurality of different time points may be distinguished from one another.

3. The ultrasound image display apparatus (3000) of claim 1,
wherein the respective pieces of ultrasound data for the plurality of time points comprise first ultrasound data acquired by scanning the object at a first time point, and second ultrasound data acquired by scanning the object at a second time point, and wherein the diagnosis image is an ultrasound image in which a first target image representing the at least one target based on the first ultrasound data and a second target image representing the at least one target based on the second ultrasound data are overlappingly displayed.

4. The ultrasound image display apparatus (3000) of claim 3, wherein the first target image and the second target image are distinguishable from each other when displayed in the diagnosis image.

5. The ultrasound image display apparatus (3000) of claim 3, wherein a difference between the first target image and the second target image is highlighted in the diagnosis image.

6. The ultrasound image display apparatus (3000) of claim 3,
wherein the image processor (3100) acquires a first size of the at least one target based on the first ultrasound data and a second size of the at least one target based on the second ultrasound data, and
wherein the display (3200) further displays at least one selected from size information for the first size, size information for the second size, and information representing a size change of the at least one target, which are acquired based on the first size and the second size.

7. The ultrasound image display apparatus (3000) of claim 1, wherein the object is an ovary, and the at least one target comprises a follicle, in which ovulation is induced, from among follicles included in the ovary.

8. The ultrasound image display apparatus (3000) of claim 1, wherein
the object is a part of the abdomen including a womb, and
the at least one target comprises at least one tumor generated in at least one part of within a womb and an outside womb.

9. The ultrasound image display apparatus (3000) of claim 1, wherein
the screen image comprises respective ultrasound images for a plurality of time points, which is obtained based on the respective pieces of ultrasound data for the plurality of time points, and
the respective ultrasound images for the plurality of time points are arranged in the order of time points at which the object is scanned.

10. The ultrasound image display apparatus (3000) of claim 1, wherein the first information represents a change in at least one selected from the size, position, and number of the at least one target at the plurality of different time points.

11. The ultrasound image display apparatus (3000) of claim 1, wherein the image screen further comprises target change numeric information that numerically represents a change in at least one selected from the size, position, and number of the at least one target.

12. The ultrasound image display apparatus (3000) of claim 11, wherein the target change numeric information comprises a value of at least one selected from an area, a volume, a long-axis length, a short-axis length, a radius, a diameter, and a circumference that represent the size of the at least one target.

13. The ultrasound image display apparatus (3000) of claim 1, further comprising a communicator which receives the respective pieces of ultrasound data for the plurality of time points from an external source.

14. A method (2300) of displaying an ultrasound image, the method (2300) comprising:
acquiring (S2310) respective pieces of ultrasound data for a plurality of time points, which represent an object including at least one target at a plurality of different time points;
acquiring respective ultrasound images for a plurality of time points based on the respective pieces of ultrasound data for the plurality of time points;
acquiring (S2320) first information representing a change in the at least one target at the plurality of different time points, based on a correspondence between the respective pieces of ultrasound data for the plurality of time points;
setting a weight for each of the respective ultrasound images for the plurality of time points;
generating a diagnosis image that shows the first information and is an image in which respective ultrasound images for the plurality of time points for each of which the weight is set are overlapped with one another and displayed; and
displaying (S2330) a screen image including the diagnosis image,
wherein the weight is a value that is applied to each of the respective ultrasound images for the plurality of time points so that each of the respective ultrasound images for the plurality of time points is more distinctly displayed or less distinctly displayed on the diagnosis image.

## Patentansprüche

1. Ultraschallbildanzeigevorrichtung (3000), die umfasst:
eine Bildverarbeitungseinrichtung (3100), die jeweils Ultraschalldaten für eine Mehrzahl von Zeitpunkten erfasst, wobei die Daten ein Objekt umfassend wenigstens ein Ziel zu einer Mehrzahl von unterschiedlichen Zeitpunkten repräsentieren, und die erste Informationen erfasst, welche eine Änderung bei dem wenigstens einen Ziel zu der Mehrzahl von unterschiedlichen Zeitpunkten repräsentiert, basierend auf einer Übereinstimmung zwischen den jeweiligen Ultraschalldaten für die Mehrzahl von Zeitpunkten; und
eine Anzeige (3200), die ein Bildschirmbild, das ein Diagnosebild umfasst und die ersten Informationen zeigt, anzeigt,
wobei die Bildverarbeitungseinrichtung (3100) jeweils Ultraschallbilder für eine Mehrzahl von Zeitpunkten basierend auf den jeweiligen Ultraschalldaten für die Mehrzahl von Zeitpunkten erfasst und eine Gewichtung für jedes der jeweiligen Ultraschallbilder für die Mehrzahl von Zeitpunkten festlegt, und das Diagnosebild erzeugt, bei dem es sich um ein Bild handelt, bei dem die jeweiligen Ultraschallbilder für die Mehrzahl von Zeitpunkten, bei denen die Gewichtung jeweils festgelegt ist, miteinander überlappt und dann angezeigt werden, und
wobei es sich bei der Gewichtung um einen Wert handelt, der bei jedem der jeweiligen Ultraschallbilder für die Mehrzahl von Zeitpunkten angewendet wird, so dass jedes der jeweiligen Ultraschallbilder für die Mehrzahl von Zeitpunkten deutlicher oder weniger deutlich in dem Diagnosebild angezeigt wird.

2. Ultraschallbildanzeigevorrichtung (3000) gemäß Anspruch 1, wobei es sich bei dem Diagnosebild um ein Ultraschallbild handelt, das derart angezeigt wird, dass die Zustände des wenigstens einen Ziels zu der Mehrzahl von unterschiedlichen Zeitpunkten voneinander unterschieden werden können.

3. Ultraschallbildanzeigevorrichtung (3000) gemäß Anspruch 1,
wobei die jeweiligen Ultraschalldaten für die Mehrzahl von Zeitpunkten erste Ultraschalldaten, die durch Abtasten des Objekts zu einem ersten Zeitpunkt erfasst worden sind, und zweite Ultraschalldaten, die durch Abtasten des Objekts zu einem zweiten Zeitpunkt erfasst worden sind, umfassen, und wobei es sich bei dem Diagnosebild um ein Ultraschallbild handelt, bei dem ein erstes Bild des Ziels, welches das wenigstens eine Ziel basierend auf den ersten Ultraschalldaten repräsentiert, und ein zweites Bild des Ziels, welches das wenigstens eine Ziel basierend auf den zweiten Ultraschalldaten repräsentiert, überlappend angezeigt werden.

4. Ultraschallbildanzeigevorrichtung (3000) gemäß Anspruch 3, wobei das erste Bild des Ziels und das zweite Bild des Ziels voneinander unterschieden werden können, wenn sie in dem Diagnosebild angezeigt werden.

5. Ultraschallbildanzeigevorrichtung (3000) gemäß Anspruch 3, wobei ein Unterschied zwischen dem ersten Bild des Ziels und dem zweiten Bild des Ziels in dem Diagnosebild hervorgehoben wird.

6. Ultraschallbildanzeigevorrichtung (3000) gemäß Anspruch 3,
wobei die Bildverarbeitungseinrichtung (3100) eine erste Größe des wenigstens einen Ziels basierend auf den ersten Ultraschalldaten und eine zweite Größe des wenigstens einen Ziels basierend auf den zweiten Ultraschalldaten erfasst, und
wobei die Anzeige (3200) weiterhin Größeninformationen für die erste Größe, Größeninformationen für die zweite Größe und/oder Informationen, die eine Größenveränderung des wenigstens einen Ziels repräsentieren, welche basierend auf der ersten Größe und der zweiten Größe erfasst worden sind, anzeigt.

7. Ultraschallbildanzeigevorrichtung (3000) gemäß Anspruch 1, wobei es sich bei dem Objekt um einen Eierstock handelt und das wenigstens eine Ziel einen Follikel aus einer Menge von in dem Eierstock vorhandenen Follikeln, in dem ein Eisprung ausgelöst wird, umfasst.

8. Ultraschallbildanzeigevorrichtung (3000) gemäß Anspruch 1, wobei
es sich bei dem Objekt um einen Teil des Abdomens einschließlich der Gebärmutter handelt, und es sich bei dem wenigstens einen Ziel um wenigstens einen Tumor handelt, der in einem Teil im Inneren der Gebärmutter oder außerhalb der Gebärmutter gewachsen ist.

9. Ultraschallbildanzeigevorrichtung (3000) gemäß Anspruch 1, wobei
das Bildschirmbild jeweilige Ultraschallbilder für eine Mehrzahl von Zeitpunkten umfasst, wobei das Bildschirmbild basierend auf den jeweiligen Sätzen von Ultraschalldaten für die Mehrzahl von Zeitpunkten erhalten worden ist, und
die jeweiligen Ultraschallbilder für die Mehrzahl von Zeitpunkten nach der Reihenfolge der Zeitpunkte, an denen das Objekt abgetastet worden ist, angeordnet sind.

10. Ultraschallbildanzeigevorrichtung (3000) gemäß Anspruch 1, wobei die ersten Informationen eine Veränderung bei der Größe, der Position und/oder der Anzahl des wenigstens einen Ziels für die Mehrzahl von unterschiedlichen Zeitpunkten repräsentieren.

11. Ultraschallbildanzeigevorrichtung (3000) gemäß Anspruch 1, wobei das Bildschirmbild weiterhin umfasst: numerische Informationen über eine Veränderung des Ziels, die eine Veränderung bei der Größe, der Position und/oder der Anzahl des wenigstens einen Ziels numerisch repräsentieren.

12. Ultraschallbildanzeigevorrichtung (3000) gemäß Anspruch 11, wobei die numerischen Informationen über eine Veränderung des Ziels umfassen: einen Wert eines Bereichs, eines Volumens, einer Länge der langen Achse, einer Länge der kurzen Achse, eines Radius, eines Durchmessers und/oder eines Umfangs, welche die Größe des wenigstens einen Ziels repräsentieren.

13. Ultraschallbildanzeigevorrichtung (3000) gemäß Anspruch 1, die weiterhin eine Kommunikationseinrichtung umfasst, welche die jeweiligen Ultraschalldaten für die Mehrzahl von Zeitpunkten von einer externen Quelle empfängt.

14. Verfahren (2300) zum Abtasten eines Ultraschallbilds, wobei das Verfahren (2300) umfasst:
Erfassen (S2310) von jeweiligen Ultraschalldaten für eine Mehrzahl von Zeitpunkten, wobei die Daten ein Objekt umfassend wenigstens ein Ziel zu einer Mehrzahl von unterschiedlichen Zeitpunkten repräsentieren;
Erfassen jeweiliger Ultraschallbilder für eine Mehrzahl von Zeitpunkten basierend auf den jeweiligen Ultraschalldaten für die Mehrzahl von Zeitpunkten;
Erfassen (S2320) von ersten Informationen, welche eine Änderung bei dem wenigstens einen Ziel zu der Mehrzahl von unterschiedlichen Zeitpunkten repräsentieren, basierend auf einer Übereinstimmung zwischen den jeweiligen Ultraschalldaten für die Mehrzahl von Zeitpunkten;
Festlegen einer Gewichtung für jedes der jeweiligen Ultraschallbilder für die Mehrzahl von Zeitpunkten;
Erzeugen eines Diagnosebilds, das die ersten Informationen zeigt, und bei dem es sich um ein Bild handelt, bei dem die jeweiligen Ultraschallbilder für die Mehrzahl von Zeitpunkten, bei denen die Gewichtung jeweils festgelegt ist, miteinander überlappt und dann angezeigt werden; und
Anzeigen (S2330) eines Bildschirmbilds, welches das Diagnosebild beinhaltet, wobei es sich bei der Gewichtung um einen Wert handelt, der bei jedem der jeweiligen Ultraschallbilder für die Mehrzahl von Zeitpunkten angewendet wird, so dass jedes der jeweiligen Ultraschallbilder für die Mehrzahl von Zeitpunkten deutlicher oder weniger deutlich in dem Diagnosebild angezeigt wird.

## Revendications

1. Appareil d'affichage d'image échographique (3000) comprenant :
un processeur d'images (3100) qui acquiert des données échographiques respectives relatives à une pluralité d'instants représentant un objet comportant au moins une cible à une pluralité d'instants différents, et qui acquiert des premières informations représentatives d'un changement concernant l'au moins une cible à la pluralité d'instants différents, compte tenu d'une correspondance entre les données échographiques respectives relatives à la pluralité d'instants, et
un dispositif d'affichage (3200) qui affiche une image à l'écran comportant une image diagnostique dans laquelle sont représentées les premières informations ;
ledit processeur d'images (3100) acquérant des images échographiques respectives relatives à une pluralité d'instants compte tenu des données échographiques respectives relatives à la pluralité d'instants et établit une pondération pour chacune des images échographiques respectives relatives à la pluralité d'instants, et produit l'image diagnostique consistant en une image dans laquelle les images échographiques respectives relatives à la pluralité d'instants pour lesquels la pondération a été établie sont superposées les unes aux autres et affichées, et
ladite pondération étant une valeur appliquée à chacune des images échographiques respectives relatives à la pluralité d'instants de manière que chacune des images échographiques respectives relatives à la pluralité d'instants soit plus distinctement ou moins distinctement affichée sur l'image diagnostique.

2. Appareil d'affichage d'image échographique (3000) selon la revendication 1, dans lequel l'image diagnostique est une image échographique affichée de manière que les états de l'au moins une cible à la pluralité d'instants différents puissent être différenciés les uns des autres.

3. Appareil d'affichage d'image échographique (3000) selon la revendication 1,
dans lequel les données échographiques respectives relatives à la pluralité d'instants comprennent des premières données échographiques acquises par échographie de l'objet à un premier instant, et des secondes données échographiques acquises par échographie de l'objet à un second instant, et dans lequel l'image diagnostique est une image échographique dans laquelle une première image de la cible représentant l'au moins une cible compte tenu des premières données échographiques et une seconde image de la cible représentant l'au moins une cible compte tenu des secondes données échographiques sont affichées superposées.

4. Appareil d'affichage d'image échographique (3000) selon la revendication 3, dans lequel la première image de la cible et la seconde image de la cible sont différentiables l'une de l'autre lorsqu'elles sont affichées dans l'image diagnostique.

5. Appareil d'affichage d'image échographique (3000) selon la revendication 3, dans lequel une différence entre la première image de la cible et la seconde image de la cible est mise en évidence dans l'image diagnostique.

6. Appareil d'affichage d'image échographique (3000) selon la revendication 3,
dans lequel le processeur d'images (3100) acquiert une première taille de l'au moins une cible compte tenu des premières données échographiques et une seconde taille de l'au moins une cible compte tenu des secondes données échographiques, et
dans lequel le dispositif d'affichage (3200) affiche en outre des informations de taille relatives à la première taille, des informations de taille relatives à la seconde taille, et/ou des informations représentant une modification de la taille de l'au moins une cible, lesquelles informations sont acquises compte tenu de la première taille et de la seconde taille.

7. Appareil d'affichage d'image échographique (3000) selon la revendication 1, dans lequel l'objet est un ovaire, et l'au moins une cible comprend un follicule, dans lequel l'ovulation est induite, parmi les follicules renfermés par l'ovaire.

8. Appareil d'affichage d'image échographique (3000) selon la revendication 1, dans lequel l'objet fait partie de l'abdomen, y compris l'utérus, et
l'au moins une cible comprend au moins une tumeur produite dans au moins une partie située à intérieur de l'utérus ou sur l'extérieur de l'utérus.

9. Appareil d'affichage d'image échographique (3000) selon la revendication 1, dans lequel l'image à l'écran comprend des images échographiques respectives relatives à une pluralité d'instants, ladite image à l'écran étant obtenue compte tenu des données échographiques respectives relatives à la pluralité d'instants, et
les images échographiques respectives relatives à la pluralité d'instants sont agencées dans l'ordre des instants auxquels l'objet a été échographié.

10. Appareil d'affichage d'image échographique (3000) selon la revendication 1, dans lequel les premières informations représentent un changement concernant la taille, l'emplacement et/ou le nombre de l'au moins une cible à la pluralité d'instants différents.

11. Appareil d'affichage d'image échographique (3000) selon la revendication 1, dans lequel l'image à l'écran comprend en outre des informations chiffrées indicatives du changement de la cible qui représentent par des chiffres un changement concernant la taille, l'emplacement et/ou le nombre de l'au moins une cible.

12. Appareil d'affichage d'image échographique (3000) selon la revendication 11, dans lequel les informations chiffrées indicatives du changement de la cible comprennent une valeur concernant une zone, un volume, une longueur de l'axe long, une longueur de l'axe court, un rayon, un diamètre et/ou une circonférence représentant la taille de l'au moins une cible.

13. Appareil d'affichage d'image échographique (3000) selon la revendication 1, comprenant en outre un appareil de communication qui reçoit les données échographiques respectives relatives à la pluralité d'instants en provenance d'une source externe.

14. Procédé (2300) d'affichage d'image échographique, le procédé (2300) comprenant :
l'acquisition (S2310) de données échographiques respectives relatives à une pluralité d'instants représentant un objet comportant au moins une cible à une pluralité d'instants différents,
l'acquisition d'images échographiques respectives relatives à une pluralité d'instants compte tenu des données échographiques respectives relatives à la pluralité d'instants,
l'acquisition (S2320) de premières informations représentatives d'un changement concernant l'au moins une cible à la pluralité d'instants différents, compte tenu d'une correspondance entre les données échographiques respectives relatives à la pluralité d'instants,
l'établissement d'une pondération pour chacune des images échographiques respectives relatives à la pluralité d'instants,
la production d'une image diagnostique dans laquelle sont représentées les premières informations et consistant en une image dans laquelle les images échographiques respectives relatives à la pluralité d'instants pour lesquels la pondération a été établie sont superposées les unes aux autres et affichées, et
l'affichage (S2330) d'une image à l'écran comportant l'image diagnostique ;
ladite pondération étant une valeur appliquée à chacune des images échographiques respectives relatives à la pluralité d'instants de manière que chacune des images échographiques respectives relatives à la pluralité d'instants soit plus distinctement ou moins distinctement affichée sur l'image diagnostique.
